# EUROPÄISCHE PATENTANMELDUNG

(11) **EP 2 327 700 A1**
(43) Veröffentlichungstag der Anmeldung: **01.06.2011**
(21) Anmeldenummer: 09014538.4
(22) Anmeldetag: 21.11.2009
(51) Int. Cl.: C07D 251/22, C07D 401/12, C07D 405/12, C07D 409/12, A01N 43/66

(54) **Dialkyl-Triazinamine und ihre Verwendung zur Bekämpfung unerwünschten Pflanzenwachstums**

(71) Anmelder: Bayer CropScience AG, 40789 Monheim (DE)
(72) Erfinder: Minn, Klemens, Dr., D-65795 Hattersheim (DE); Dietrich, Hansjörg, Dr., D-65835 Liederbach (DE); Rosinger, Christopher, Dr., D-65719 Hofheim (DE); Feucht, Dieter, Dr., D-65760 Eschborn (DE); Häuser-Hahn, Isolde, Dr., D-51375 Leverkusen (DE); Dittgen, Jan, Dr., D-60316 Frankfurt a. M. (DE)

(57) **Zusammenfassung**

Beschrieben werden Verbindungen der Formel (I) und deren agrochemisch verträglichen Salze 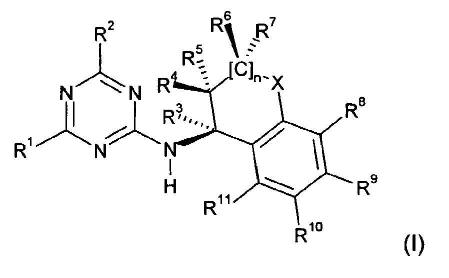 und deren Verwendung im Bereich des Pflanzenschutzes.

In dieser Formel (I) stehen R¹ bis und R¹¹ für Reste wie Wasserstoff, organische Reste wie Alkyl, und andere Reste wie Halogen. Die Laufzahl n steht für die Werte 1, 2 oder 3 und X steht für eine Bindung oder Sauerstoff und Schwefel.

## Beschreibung

Die Erfindung betrifft das technische Gebiet der Pflanzenschutzmittel, insbesondere das der Herbizide zur selektiven Bekämpfung von Unkräutern und Ungräsern in Nutzpflanzenkulturen.

Speziell betrifft sie Dialkyl-Triazinamine, die aminseitig mit einem bicyclischen Fragment verknüpft sind, Verfahren zu deren Herstellung sowie deren Verwendung zur Bekämpfung von Schadpflanzen.

Aus dem Stand der Technik sind Dialkyl-Triazinamine bekannt, welche eine herbizide Wirkung aufweisen. So beschreibt beispielsweise die JP 246528/99, JP2001/247411 A, WO 01/90085 A2, WO 01/92236 A1 bestimmte Dialkyl-Triazinamine und deren Anwendung im Bereich des Pflanzenschutzes. Die bisher beschriebenen Derivate tragen ein offenkettiges Amin. Die Anwendung der Derivate dieses Typs als selektive Herbizide zur Schadpflanzenbekämpfung oder als Pflanzenwachstumsregulatoren in verschiedenen Nutzpflanzenkulturen erfordert jedoch häufig eine unzeitgemäße Aufwandmenge oder führt zu unerwünschten Schädigungen der Nutzpflanzen. Zudem ist die Anwendung der Wirkstoffe in vielen Fällen wegen verhältnismäßig hoher Herstellkosten unwirtschaftlich.

Es ist deshalb wünschenswert, alternative chemische Wirkstoffe auf Basis von Dialkyl-Triazinamine-Derivaten bereitzustellen, die als Herbizide oder Pflanzenwachstumsregulatoren eingesetzt werden können und mit welchen bestimmte Vorteile im Vergleich zu aus dem Stand der Technik bekannten Systemen verbunden sind.

Damit ergibt sich im Allgemeinen als Aufgabe der vorliegenden Erfindung, alternative Dialkyl-Triazinamine-Derivate bereitzustellen, welche als Herbizide oder Pflanzenwachstumsregulatoren, insbesondere mit einer zufriedenstellenden herbiziden Wirkung gegen Schadpflanzen, mit einem breiten Spektrum gegenüber Schadpflanzen und/oder mit einer hohen Selektivität in Nutzpflanzenkulturen, eingesetzt werden können. Diese Dialkyl-Triazinamine -Derivate sollten dabei vorzugsweise ein besseres Eigenschaftsprofil, insbesondere eine bessere herbizide Wirkung gegen Schadpflanzen, ein breiteres Spektrum gegenüber Schadpflanzen und/oder eine höhere Selektivität in Nutzpflanzenkulturen, als die aus dem Stand der Technik bekannten Dialkyl-Triazinaminederivate zeigen.

Erfindungsgemäß wurden nun neue Dialkyl-Triazinamine gefunden, welche vorteilhaft als Herbizide und Pflanzenwachstumsregulatoren eingesetzt werden können. Die Verbindungen dieser Reihe zeichnen sich neben gutem Wirksamkeitsprofil und Kulturpflanzenverträglichkeit durch kostengünstige Herstellprozesse und Zugangsmöglichkeiten aus, da die erfindungsgemäßen Substanzen aus preiswerten und gut zugänglichen Vorstufen hergestellt werden können und daher auf die Verwendung teurer und schwer zugänglicher Zwischenprodukte verzichtet werden kann.

Ein Gegenstand der vorliegenden Erfindung sind Verbindungen der Formel (I) und deren agrochemisch verträglichen Salze 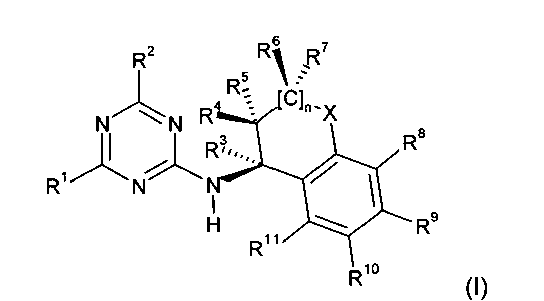 worin
- R¹ und R²: bedeuten unabhängig voneinander jeweils
(C₁-C₆)-Alkyl, (C₁-C₆)-Halogenalkyl, (C₁-C₆)-Halogenalkylcarbonyloxy, (C₁-C₆)-Alkoxycarbonyl-(C₁-C₄)-alkyl, (C₆-C₁₄)-Aryl-(C₁-C₆)-alkyl, (C₁-C₆)-Alkoxy-(C₁-C₆)-alkyl, (C₃-C₈)-Cycloalkyl, welches am Cycloalkylrest durch s Reste aus der Gruppe (C₁-C₆)-Alkyl und oder Halogen substituiert ist, (C₃-C₈)-Cycloalkyl-(C₁-C₆)-alkyl, (C₃-C₈)-Cycloalkyl-(C₁-C₆)-haloalkyl, (C₃-C₈)-Cycloalkenyl, (C₃-C₈)-Cycloalkenyl-(C₁-C₆)-alkyl, (C₃-C₈)-Cycloalkenyl-(C₁-C₆)-halogenalkyl, Hydroxy-(C₁-C₆)-alkyl oder Cyano-(C₁-C₆)-alkyl;
- R³: bedeutet Wasserstoff, (C₁-C₆)-Aolky) oder (C₁-C₆)-Haloalkyl;
- R⁴ und R⁵: bedeuten unabhängig voneinander jeweils
Wasserstoff, (C₁-C₆)-Alkyl, (C₁-C₆)-Haloalkyl, Hydroxy, (C₁-C₆)-Alkoxy, (C₁-C₆)-Halogenalkoxy, oder Benzyl, das im Phenylring durch eine Methylgruppe substituiert ist, oder die Reste R⁴ und R⁵ bilden eine (C₂-C₇)-Alkylengruppe, die ein oder mehrere Sauerstoff- und/oder Schwefelatome enthalten können, wobei die (C₂-C₇)-Alkylengruppe durch s Halogenatome substituiert ist und die jeweilgen Halogensubstituenten gleich oder verschieden sein können;
- R⁶ und R⁷: bedeuten unabhängig voneinander jeweils
Wasserstoff, (C₁-C₆)-Alkyl, (C₁-C₆)-Haloalkyl, (C₁-C₆)-Alkoxy, (C₁-C₆)-Halogenalkoxy, (C₆-C₁₄)-Aryl, (C₆-C₁₄)-Aryloxy, (C₆-C₁₄)-Arylcarbonyl und (C₆-C₁₄)-Aryloxycarbonyl; oder die Reste R⁶ und R⁷ bilden eine (C₂-C₇)-Alkylengruppe, die ein oder mehrere Sauerstoff- und/oder Schwefelatome enthalten können, wobei die (C₂-C₇)-Alkylengruppe durch s Halogenatome substituiert ist und die jeweilgen Halogensubstituenten gleich oder verschieden sein können;
R⁸, R⁹, R¹⁰ und R¹¹ bedeuten unabhängig voneinander jeweils
Wasserstoff, Halogen, Cyano, (C₁-C₆)-Alkyl, (C₁-C₆)-Alkylcarbonyl, (C₁-C₆)-Alkyloxycarbonyl, (C₁-C₆)-Alkylaminocarbonyl, (C₁-C₆)-di-Alkylaminocarbonyl, (C₁-C₆)-Haloalkyl, (C₁-C₆)-Alkoxy, (C₁-C₆)-Halogenalkoxy, (C₂-C₆)-Alkinyl, (C₂-C₆)-Halogenalkinyl, (C₂-C₆)-Alkinylcarbonyl, (C₂-C₆)-Haloalkinylcarbonyl, (C₂-C₆)-Alkinyloxy, (C₂-C₆)-Haloalkinyloxy, (C₂-C₆)-Alkinyloxycarbonyl, (C₂-C₆)-Halogenalkinyloxycarbonyl, (C₁-C₆)-di-Alkylamino, Aminocarbonyl, Cyano-(C₁-C₆)-alkyl, (C₁-C₆)-Alkyloxy-(C₁-C₆)-alkyl, (C₁-C₆)-Alkyl-(C₂-C₆)-alkinyl, Methoxymethyl-(C₂-C₆)-alkinyl, Trimethylsilyl-(C₂-C₆)-alkinyl oder Nitro;
- R¹², R¹³ und R¹⁴: bedeuten unabhängig voneinander jeweils Wasserstoff, (C₁-C₆)-Alkyl oder (C₁-C₆)-Haloalkyl;
- X: bedeutet eine Bindung, CH₂, Sauerstoff, Schwefel, Carbonyl, NH, CR¹²R¹³ oder NR¹⁴;
- n: bedeutet 1, 2 oder 3, und
- s: bedeutet 0, 1, 2, 3, 4 oder 5.

Erfindungsgemäß wurde gefunden, dass diese Verbindungen ein gutes Wirksamkeitsprofil und eine hohe Kulturpflanzenverträglichkeit aufweisen.

Bevorzugt sind Verbindungen der Formel (I), worin
- R¹ und R²: bedeuten unabhängig voneinander jeweils
(C₁-C₆)-Alkyl, (C₁-C₆)-Halogenalkyl, (C₁-C₆)-Alkoxycarbonyl-(C₁-C₄)-alkyl, (C₁-C₆)-Alkoxy-(C₁-C₆)-alkyl oder (C₃-C₈)-Cycloalkyl;
- R³: bedeutet Wasserstoff oder Methyl;
- R⁴ und R⁵: bedeuten unabhängig voneinander jeweils
Wasserstoff, (C₁-C₆)-Alkyl, (C₁-C₆)-Alkoxy oder Benzyl, das im Phenylring durch eine Methylgruppe substituiert ist, oder die Reste R⁴ und R⁵ bilden eine (C₂-C₇)-Alkylengruppe, die ein oder mehrere Sauerstoff- und/oder Schwefelatome enthalten können, wobei die (C₂-C₇)-Alkylengruppe durch s Halogenatome substituiert ist und die jeweilgen Halogensubstituenten gleich oder verschieden sein können;
- R⁶ und R⁷: bedeuten unabhängig voneinander jeweils
Wasserstoff oder (C₁-C₆)-Alkyl;
- R⁸ und R⁹: bedeuten unabhängig voneinander jeweils
Wasserstoff, (C₁-C₆)-Alkyl, (C₁-C₆)-Alkoxy, Cyano oder Halogen;
- R¹⁰: bedeutet Wasserstoff, Halogen, Cyano, (C₁-C₆)-Alkyl, (C₁-C₆)-Alkylcarbonyl, (C₁-C₆)-Alkoxy, (C₂-C₆)-Alkinyl, (C₁-C₆)-di-Alkylamino, Aminocarbonyl, (C₁-C₆)-Alkylaminocarbonyl, (C₁-C₆)-di-Alkylaminocarbonyl, Cyano-(C₁-C₆)-alkyl, (C₁-C₆)-Alkyloxy-(C₁-C₆)-alkyl, (C₁-C₆)-Halogenalkoxy, (C₁-C₆)-Alkyl-(C₂-C₆)-alkinyl, Methoxymethyl-(C₂-C₆)-alkinyl, Trimethylsilyl-(C₂-C₆)-alkinyl oder Nitro;
- R¹¹: bedeutet Wasserstoff oder (C₁-C₆)-Alkyl;
- R¹², R¹³ und R¹⁴: bedeuten unabhängig voneinander jeweils Wasserstoff oder (C₁-C₆)-Alkyl;
- X: bedeutet eine Bindung, CH₂, Sauerstoff, Schwefel, CR¹²R¹³ oder NR¹⁴;
- n: bedeutet 1, 2 oder 3, und
- s: bedeutet 0, 1, 2 oder 3.

Besonders bevorzugt sind Verbindungen der Formel (I), worin
- R¹: bedeutet Methyl, Ethyl, Propyl, i-Propyl, c-Propyl, t-Butyl oder Trichlormethyl;
- R²: bedeutet Methyl, Ethyl, Propyl, i-Propyl, c-Propyl, t-Butyl, Trihalogenmethyl, Dihalogenmethyl, Halogenethyl, Methoxymethyl oder Methoxycarbonylmethyl;
- R³: bedeutet Wasserstoff oder Methyl;
- R⁴: bedeutet
Wasserstoff, Methyl, Ethyl, Methoxy oder Benzyl, das im Phenylring durch eine Methylgruppe substituiert ist,
- R⁵: bedeutet Wasserstoff, Methyl oder Ethyl,
oder die Reste R⁴ und R⁵ bilden die Gruppe CH₂CH₂,
- R⁶ und R⁷: bedeuten unabhängig voneinander jeweils
bedeutet Wasserstoff, Methyl oder Ethyl,
- R⁸: bedeutet Wasserstoff, Methyl, Ethyl, Halogen, Cyano oder Methoxy;
- R⁹: bedeutet Wasserstoff, Halogen, Cyano oder Methoxy;
- R¹⁰: bedeutet Wasserstoff, Halogen, Cyano, (C₁-C₆)-Alkyl, (C₁-C₆)-Alkylcarbonyl, (C₁-C₆)-Alkoxy, (C₂-C₆)-Alkinyl, (C₁-C₂)-di-Alkylamino, Aminocarbonyl, (C₁-C₂)-Alkylaminocarbonyl, (C₁-C₆)-di-Alkylaminocarbonyl, Cyano-(C₁-C₆)-alkyl, (C₁-C₂)-Alkyloxy-(C₁-C₂)-alkyl, (C₁-C₆)-Halogenalkoxy, (C₁-C₆)-Alkyl-(C₂-C₄)-alkinyl, Methoxymethyl-(C₂-C₄)-alkinyl, Trimethylsilyl-(C₂-C₄)-alkinyl oder Nitro;
- R¹¹: bedeutet Wasserstoff Methyl;
- R¹² und R¹³: bedeuten unabhängig voneinander jeweils Wasserstoff, Methyl oder Ethyl;
- R¹⁴: bedeutet Methyl oder Ethyl;
- X: bedeutet eine Bindung, CH₂, Sauerstoff, Schwefel, CR¹² R¹³ oder NR¹⁴;
- n: bedeutet 1, und
- s: bedeutet 0, 1, 2 oder 3.

Im Rahmen der vorliegenden Erfindung sind von der Verbindung der Formel (I) auch Verbindungen umfasst, die durch Deprotonierung, Protonierung, Alkylierung oder Oxidation an einem Stickstoffatom quaterniert sind. Insbesondere sind diesbzüglich die entsprechenden N-Oxide zu nennnen.

Die Verbindungen der Formel (I) können Salze bilden. Salzbildung kann durch Einwirkung einer Base auf solche Verbindungen der Formel (I) erfolgen, die ein acides Wasserstoffatom tragen. Geeignete Basen sind beispielsweise organische Amine, wie Trialkylamine, Morpholin, Piperidin oder Pyridin sowie Ammonium-, Alkalioder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze, zum Beispiel mit Kationen der Formel [NRR'R"R''']⁺, worin R bis R''' jeweils unabhängig voneinander einen organischen Rest, insbesondere Alkyl, Aryl, Aralkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxonium-salze.

Die Verbindungen der Formel (I) können durch Anlagerung einer geeigneten anorganischen oder organischen Säure, wie beispielsweise Mineralsäuren, wie beispielsweise HCl, HBr, H₂SO₄, H₃PO₄ oder HNO₃, oder organische Säuren, z. B. Carbonsäuren, wie Ameisensäure, Essigsäure, Propionsäure, Oxalsäure, Milchsäure oder Salicylsäure oder Sulfonsäuren, wie zum Beispiel p-Toluolsulfonsäure, an eine basische Gruppe, wie z.B. Amino, Alkylamino, Dialkylamino, Piperidino, Morpholino oder Pyridino, Salze bilden. Diese Salze enthalten dann die konjugierte Base der Säure als Anion.

Geeignete Substituenten, die in deprotonierter Form, wie z.B. Sulfonsäuren oder Carbonsäuren, vorliegen, können innere Salze mit ihrerseits protonierbaren Gruppen, wie Aminogruppen bilden.

Im Folgenden werden die Verbindungen der Formel (I) und ihre Salze auch kurz als erfindungsgemäß verwendete oder erfindungsgemäße "Verbindungen (I)" bezeichnet.

In der allgemeinen Formel (I) und allen übrigen Formeln in der vorliegenden Erfindung können die Reste Alkyl, Alkoxy, Haloalkyl, Haloalkoxy, Alkylamino, Alkylthio, Haloalkylthio, sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z. B. mit 1 bis 6 Kohlenstoffatomen, insbesondere 1 bis 4 Kohlenstoffatomen, bzw. bei ungesättigten Gruppen mit 2 bis 6 Kohlenstoffatomen, insbesondere 2 bis 4 Kohlenstoffatomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexy und 1,4-Dimethylpentyl; Alkenyl- und Alkinylreste haben die Bedeutung der den Alkylresten entsprechenden möglichen ungesättigten Reste; wobei mindestens eine Doppelbindung bzw. Dreifachbindung, vorzugsweise eine Doppelbindung bzw. Dreifachbindung enthalten ist. Alkenyl bedeutet z.B. Vinyl, Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Ethinyl, Propargyl, But-2-in-1-yl, But-3-in-1-yl und 1-Methyl-but-3-in-1-yl.

Cycloalkyl-Gruppen sind z. B. Cyclopropyl, Cyclobutyl, Cyclopentyl, Cyclohexyl, Cycloheptyl und Cyclooctyl. Die Cycloalkyl Gruppen können in bi- oder tri-cyclischer Form vorkommen.

Wenn Haloalkylgruppen und Haloalkylreste von Haloalkoxy, Haloalkylthio, Haloalkenyl, Haloalkinyl u.a. angegeben sind, sind bei diesen Resten die niederen Kohlenstoffgerüste, z. B. mit 1 bis 6 C-Atomen oder 2 bis 6, insbesondere 1 bis 4 C-Atomen oder bevorzugt 2 bis 4 C-Atomen, sowie die entsprechenden ungesättigten und/oder substituierten Reste im Kohlenstoffgerüst jeweils geradkettig oder verzweigt. Beispiele sind Difluormethyl, 2,2,2-Trifluorethyl, Trifluorallyl, 1-Chlorprop-1-yl-3-yl.

Alkylen-Gruppen sind bei diesen Resten die niederen Kohlenstoffgerüste, z. B. mit 1 bis 10 C-Atomen, insbesondere 1 bis 6 C-Atomen oder bevorzugt 2 bis 4 C-Atomen, sowie die entsprechenden ungesättigten und/oder substituierten Reste im Kohlenstoffgerüst, die jeweils geradkettig oder verzweigt sein können. Beispiele sind Methylen, Ethylen, n- und i- Propylen und n-, s-, i-, t-Butylen.

Hydroxyalkylgruppen sind bei diesen Resten die niederen Kohlenstoffgerüste, z. B. mit 1 bis 6 C-Atomen, insbesondere 1 bis 4 C-Atomen, sowie die entsprechenden ungesättigten und/oder substituierten Resten im Kohlenstoffgerüst, die jeweils geradkettig oder verzweigt sein können. Beispiele hierzu sind 1,2-Dihydroxyethyl und 3-Hydroxypropyl.

Halogen bedeutet Fluor, Chlor, Brom oder Iod. Haloalkyl, -alkenyl und -alkinyl bedeuten durch Halogen, vorzugsweise durch Fluor, Chlor oder Brom, insbesondere durch Fluor und/oder Chlor, teilweise oder vollständig substituiertes Alkyl, Alkenyl bzw. Alkinyl, z.B. Monohaloalkyl (= Monohalogenalkyl), Perhaloalkyl, CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; entsprechendes gilt für Haloalkenyl und andere durch Halogen substituierte Reste.

Aryl bedeutet ein mono-, bi- oder polycyclisches aromatisches System, beispielsweise Phenyl oder Naphthyl, vorzugsweise Phenyl.

Vor allem aus den Gründen der höheren herbiziden Wirkung, besseren Selektivität und/oder besseren Herstellbarkeit sind erfindungsgemäße Verbindungen der allgemeinen Formel (I) oder deren agrochemischen Salze oder quartären N-Derivate von besonderem Interesse, worin einzelne Reste eine der bereits genannten oder im Folgenden genannten bevorzugten Bedeutungen haben, oder insbesondere solche, worin eine oder mehrere der bereits genannten oder im folgenden genannten bevorzugten Bedeutungen kombiniert auftreten.

Die oben angeführten allgemeinen oder in Vorzugsbereichen angeführten Restedefinitionen gelten sowohl für die Endprodukte der allgemeinen Formel (I) als auch entsprechend für die die jeweils zur Herstellung benötigten Ausgangs- und Zwischenprodukte. Diese Restedefinitionen können untereinander, als auch zwischen den angegebenen bevorzugten Bereichen vertauscht werden.

Die vorliegenden Verbindungen der allgemeinen Formel (I) weisen mindestens ein chirales Kohlenstoffatom auf, welches in der unten dargestellten Struktur durch die Kennzeichnung (*) verdeutlicht ist:

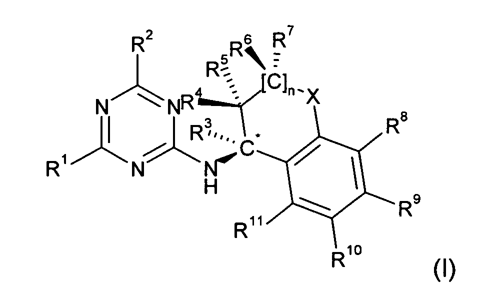

Gemäß den Regeln nach Cahn, Ingold und Prelog (CIP-Regeln) kann dieses Kohlenstoffatom sowohl eine (R)- als auch eine (S)-Konfiguration aufweisen.

Von der vorliegenden Erfindung werden Verbindungen der allgemeinen Formel (I) sowohl mit (S)- als auch mit (R)-Konfiguration erfasst, d.h., dass die vorliegende Erfindung die Verbindungen der allgemeinen Formel (I) erfasst, in welchen das betreffende Kohlenstoffatom
(1) eine (R)-Konfiguration, oder
(2) eine (S)-Konfiguration
aufweist.

Darüber hinaus werden im Rahmen der vorliegenden Erfindung auch
(3) beliebige Mischungen von Verbindungen der allgemeinen Formel (I), welche eine (R)-Konfiguation (Verbindungen der allgemeinen Formel (I-(R)) aufweisen, mit Verbindungen der allgemeinen Formel (I), welche eine (S)-Konfiguration (Verbindungen der allgemeinen Formel (I-S)) aufweisen,
erfasst, wobei eine racemische Mischung der Verbindungen der allgemeinen Formel (I) mit (R)- und (S)-Konfiguration von der vorliegenden Erfindung ebenfalls umfasst ist.

Allerdings sind im Rahmen der vorliegenden Erfindung insbesondere Verbindungen der allgemeinen Formel (I) mit (R)-Konfiguration mit einer Selektivität von 60 bis 100%, vorzugsweise 80 bis 100%, insbesondere 90 bis 100%, ganz besonders 95 bis 100%, bevorzugt, wobei die jeweilige (R)-Verbindung mit einer Enantioselektivität von jeweils mehr als 50% ee, vorzugsweise 60 bis 100% ee, insbesondere 80 bis 100% ee, ganz besonders 90 bis 100% ee, meist bevorzugt 95 bis 100% ee, bezogen auf den Gesamtgehalt an betreffender (R)-Verbindung vorliegt.

Daher betrifft die vorliegende Erfindung insbesondere Verbindungen der allgemeinen Formel (I), in welchen die stereochemische Konfiguration an dem mit (*) gekennzeichnet Kohlenstoffatom mit einer stereochemischen Reinheit von 60 bis 100 % (R), vorzugsweise 80 bis 100 % (R), insbesondere 90 bis 100 % (R), ganz besonders 95 bis 100 % (R), vorliegt.

Unter Berücksichtigung der Regel nach Cahn, Ingold und Prelog kann es an dem mit (*) gekennzeichneten Kohlenstoffatom auch zu einer Situation kommen, in welcher aufgrund der Priorität der jeweiligen Substituenten die (S)-Konfiguration am mit (*) gekennzeichneten Kohlenstoffatom bevorzugt ist. Dieses ist beispielweise dann der Fall, wenn die Reste R⁴ und/oder R⁵ einem C₁-C₆-Alkoxyrest entsprechen.

Daher sind im Rahmen der vorliegenden Erfindung insbesondere Verbindungen der allgemeinen Formel (I) bevorzugt, die in ihrer räumlichen Anordnung den jenigen Verbindungen der allgemeinen Formel (I) mit R⁴ und R⁵ =Wasserstoff mit (R)-Konfiguration entsprechen, mit einer Selektivität von 60 bis 100 %, vorzugsweise 80 bis 100 %, insbesondere 90 bis 100 %, ganz besonders 95 bis 100 %, bevorzugt, wobei die jeweilige (R)-analoge-Verbindung mit einer Enantioselektivität von jeweils mehr als 50 % ee, vorzugsweise 60 bis 100 % ee, insbesondere 80 bis 100 % ee, ganz besonders 90 bis 100 % ee, meist bevorzugt 95 bis 100 % ee, bezogen auf den Gesamtgehalt an betreffender (R)-analogen-Verbindung, vorliegt. Daher betrifft die vorliegende Erfindung insbesondere Verbindungen der allgemeinen Formel (I), in welchen die stereochemische Konfiguration an dem mit (*) gekennzeichnet Kohlenstoffatom mit einer stereochemischen Reinheit von 60 bis 100 % (R, bzw. analog-R), vorzugsweise 80 bis 100 % (R, bzw. analog-R), insbesondere 90 bis 100 % (R, bzw. analog-R), ganz besonders 95 bis 100 % (R, bzw. analog-R), vorliegt.

Insbesondere können die erfindungsgemäßen Verbindungen der allgemeinen Formel (I) noch weitere Chiralitätszentren an den mit (**) und (***) gekennzeichneten Kohlenstoffatomen aufweisen:

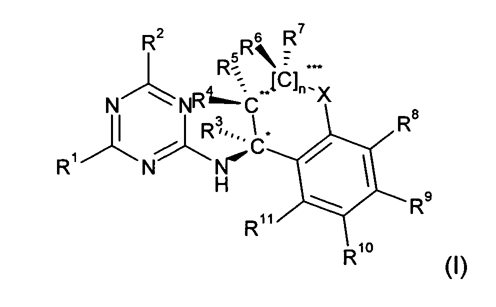

Im Rahmen der vorliegenden Erfindung sind beliebige stereochemische Konfigurationen an den mit (*), (**) und (***) gekennzeichneten Kohlenstoffatomen möglich:

| Konfiguration Kohlenstoffatom (*) | Konfiguration Kohlenstoffatom (**) | Konfiguration Kohlenstoffatom (***) |
|---|---|---|
| R | R | R |
| R | R | S |
| R | S | R |
| S | R | R |
| R | S | S |
| S | R | S |
| S | S | R |
| S | S | S |

Darüber hinaus können, je nach Wahl der jeweiligen Reste, weitere Stereoelemente in den erfindungsgemäßen Verbindungen der allgemeinen Formel (I) vorliegen.

Sind beispielsweise eine oder mehrere Alkenylgruppen vorhanden, so können Diastereomere (Z- und E-Isomere) auftreten.

Sind beispielsweise ein oder mehrere asymmetrische Kohlenstoffatome vorhanden, so können Enantiomere und Diastereomere auftreten.

Entsprechende Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangsund/oder Hilfsstoffe selektiv hergestellt werden. Die Erfindung betrifft somit auch alle Stereoisomeren, die von der allgemeinen Formel (I) umfaßt werden, jedoch nicht mit ihrer spezifischen Stereoform angegeben sind und deren Gemische.

Die Kombinationsmöglichkeiten der verschiedenen Substituenten der allgemeinen Formel (I) sind so zu verstehen, dass die allgemeinen Grundsätze des Aufbaus chemischer Verbindungen zu beachten sind, d.h. die Formel (I) nicht Verbindungen umfasst, von denen der Fachmann weiß, dass sie chemisch nicht möglich sind.

Besonders bevorzugte Beispiele der Verbindungen der allgemeinen Formel (I) sind die in nachfolgender Tabelle als I-1 bis I-259 bezeichneten Verbindungen, in denen R³ jeweils Wasserstoff bedeutet:

Die verwendeten Abkürzungen bedeuten:

| | | | |
|---|---|---|---|
| Et = Ethyl | Me = Methyl | Pr = Propyl | Bu = Butyl |
| i = iso | t = tertiär | rac = racemisch | |

Die Angaben "St C*", "St C**" und "St C***" beziehen sich auf die Stereochemie an den betreffenden Kohlenstoffatomen und geben an, ob es sich um die (R)-Konfiguration, die (S)-Konfiguration gemäß der Cahn-Ingold-Prelog Notation handelt, oder ob es sich um ein Gemisch beider Stereoisomeren in Bezug auf dieses Stereozentrum handelt (rac). Fehlt die Angabe zur Stereochemie, so ist Konfiguration am betreffenden Stereozentrum nicht bekannt.

| **Nr.** | **R¹** | **R²** | **St C*** | **R⁴** | **R⁵** | **St C**** | **R⁶** | **R⁷** | **n** | **St C^{***}** | **R⁸** | **R⁹** | **R¹⁰** | **R¹¹** | **X** |
|---|---|---|---|---|---|---|---|---|---|---|---|---|---|---|---|
| I-1 | Me | CF₃ | | H | H | | H | H | 2 | | Me | H | Me | H | - |
| I-2 | Me | CF₂H | | H | H | | H | H | 2 | | Me | H | Me | H | - |
| I-3 | Me | CF₂Cl | | H | H | | H | H | 2 | | Me | H | Me | H | - |
| I-4 | Me | CF₂H | | H | H | | H | H | 2 | | H | H | H | H | - |
| I-5 | Me | CF₃ | | H | H | | H | H | 1 | | H | H | H | H | - |
| I-6 | Me | CF₃ | | H | H | | H | H | 1 | | Me | H | H | H | - |
| I-7 | Me | CF₃ | | H | H | | H | H | 1 | | H | H | F | H | O |
| I-8 | Me | Me | | H | H | | H | H | 1 | | H | H | H | H | - |
| I-9 | Me | Me | | H | H | | H | H | 1 | | H | H | H | H | - |
| I-10 | Me | Me | | H | H | | H | H | 1 | | H | H | F | H | - |
| I-11 | Me | Me | | H | H | | H | H | 1 | | H | H | Cl | H | - |
| I-12 | Me | Me | | H | H | | H | H | 1 | | H | H | OMe | H | - |
| I-13 | Me | Me | | H | H | | H | H | 1 | | H | OMe | OMe | H | - |
| I-14 | Me | Me | | H | H | | H | H | 1 | | H | H | OEt | H | - |
| I-15 | Me | Me | | H | H | | H | H | 1 | | Me | H | H | Me | - |
| I-16 | Me | Et | | H | H | | H | H | 2 | | Me | H | Me | H | - |
| I-17 | Me | Me | | H | H | | H | H | 2 | | Me | H | Me | H | - |
| I-18 | Me | Me | | H | H | | H | H | 1 | | H | H | F | H | O |
| I-19 | Me | Et | | H | H | | H | H | 1 | | H | H | H | H | - |
| I-20 | Me | Et | | H | H | | H | H | 1 | | H | H | H | H | O |
| I-21 | Me | Et | | H | H | | H | H | 1 | | H | H | H | H | S |
| I-22 | Me | Et | | H | H | | H | H | 1 | | H | H | Me | H | S |
| I-23 | Me | Et | | Me | H | | H | H | 2 | | H | H | Me | H | - |
| I-24 | Me | Et | | OMe | H | | H | H | 2 | | H | H | H | H | - |
| I-25 | Me | Et | | CH₂-CH₂ | | | H | H | | | | H | H | H | H |
| I-26 | Me | Et | | H | H | | H | H | 2 | | H | H | I | H | - |
| I-27 | Me | Et | | H | H | | H | H | 2 | | H | H | CN | H | - |
| I-28 | Me | Et | | H | H | | H | H | 2 | | H | H | CCH | H | - |
| I-29 | Me | Et | | H | H | | H | H | 2 | | H | H | C(O)Me | H | - |
| I-30 | Me | Et | | H | H | | H | H | 2 | | H | H | C(O)NH₂ | H | - |
| I-31 | Me | Et | | H | H | | H | H | 2 | | H | H | C(O)NMe₂ | H | - |
| I-32 | Me | Et | | H | H | | H | H | 2 | | H | H | C(O)NHMe | H | - |
| I-33 | Me | i-Pr | | H | H | | H | H | 1 | | H | H | H | H | - |
| I-34 | Me | i-Pr | | H | H | | H | H | 2 | | Me | H | Me | H | - |
| I-35 | Me | i-Pr | | H | H | | H | H | 2 | | H | H | H | H | - |
| I-36 | Me | i-Pr | | H | H | | H | H | 1 | | H | H | H | H | O |
| I-37 | Me | CF₂Cl | | H | H | | H | H | 1 | | H | H | H | H | - |
| I-38 | c-Pr | CF₃ | | H | H | | H | H | 1 | | H | H | H | H | - |
| I-39 | i-Pr | CF₃ | | H | H | | H | H | 1 | | H | H | H | H | - |
| I-40 | i-Pr | CF₃ | | H | H | | H | H | 1 | | H | H | H | H | O |
| I-41 | i-Pr | CF₃ | | H | H | | H | H | 2 | | Me | Me | H | H | - |
| I-42 | Me | i-Pr | | H | H | | H | H | 2 | | Me | Me | H | H | - |
| I-43 | Me | i-Pr | | H | H | | H | H | 3 | | H | H | H | H | - |
| I-44 | CCl₃ | CHFMe <rac> | | H | H | | H | H | 1 | | H | H | H | H | - |
| I-45 | Me | CCl₃ | | H | H | | H | H | 1 | | H | H | Et | H | O |
| I-46 | Me | CCl₃ | | H | H | | Me | Me | 1 | | H | Me | H | H | O |
| I-47 | Me | CCl₃ | | CH₂-(3-Me)- Ph | H | | H | H | 1 | | H | Me | H | H | - |
| I-48 | Me | CCl₃ | | Me | H | | H | H | 1 | | H | H | Me | H | - |
| I-49 | Me | CCl₃ | R | Et | H | S | H | H | 1 | | H | H | Me | H | - |
| I-50 | Me | i-Pr | R | Me | H | S | H | H | 1 | | H | H | Me | H | - |
| I-51 | Me | t-Bu | R | Me | H | S | H | H | 1 | | H | H | Me | H | - |
| I-52 | Me | Et | R | Me | H | S | H | H | 1 | | H | H | Me | H | - |
| I-53 | Me | Et | R | H | H | | H | H | 2 | | H | H | H | H | - |
| I-54 | Me | CH₂OMe | R | Me | H | S | H | H | 1 | | H | H | Me | H | - |
| I-55 | CCl₃ | CCl₃ | R | H | H | | H | H | 2 | | H | H | H | H | - |
| I-56 | Me | CH₂OMe | R | H | H | | H | H | 1 | | H | H | Me | H | - |
| I-57 | Me | CH₂OMe | R | H | H | | H | H | 1 | | H | Me | Me | H | - |
| I-58 | Me | CH₂OMe | R | H | H | | H | H | 1 | | Me | H | Me | H | - |
| I-59 | Me | CH₂OMe | R | Me | H | S | H | H | 1 | | H | H | I | H | - |
| I-60 | Me | CH₂OMe | R | Me | H | S | H | H | 1 | | H | H | Cl | H | - |
| I-61 | Me | CH₂OMe | R | Me | H | S | H | H | 1 | | H | H | OMe | H | - |
| I-62 | Me | CH₂OMe | R | Me | H | S | H | H | 1 | | H | H | OEt | H | - |
| I-63 | Me | Me | R | Me | H | S | H | H | 1 | | H | H | Me | H | - |
| I-64 | Me | CF₂H | R | Me | H | S | H | H | 1 | | H | H | Me | H | - |
| I-65 | Me | CF₂H | R | H | H | | H | H | 1 | | H | H | H | H | - |
| I-66 | Me | CF₂Cl | R | Me | H | S | H | H | 1 | | H | H | Me | H | - |
| I-67 | Me | CF₂Cl | R | H | H | | H | H | 2 | | H | H | H | H | - |
| I-68 | Me | CCl₂H | R | H | H | | H | H | 1 | | H | H | H | H | - |
| I-69 | Me | CCl₂H | R | Me | H | S | H | H | 1 | | H | H | Me | H | - |
| I-70 | Me | CCl₃ | R | Me | H | S | H | H | 1 | | H | H | Me | H | - |
| I-71 | Me | CCl₃ | R | H | H | | H | H | 2 | | H | H | H | H | - |
| I-72 | Me | c-Pr | R | H | H | | H | H | 2 | | H | H | H | H | - |
| I-73 | Me | Et | | H | H | | H | H | 1 | | H | H | F | H | O |
| I-74 | t-Bu | CF₃ | | H | H | | H | H | 1 | | H | H | H | H | - |
| I-75 | i-Pr | CF₃ | | H | H | | H | H | 3 | | H | H | H | H | - |
| 1-76 | Me | i-Pr | R | H | H | | H | H | 2 | | H | H | H | H | - |
| I-77 | Me | t-Bu | R | H | H | | H | H | 2 | | H | H | H | H | - |
| I-78 | Me | Me | R | H | H | | H | H | 2 | | H | H | H | H | - |
| I-79 | CCl₃ | CH₂CO₂Me | R | H | H | | H | H | 2 | | H | H | H | H | - |
| I-80 | CCl₃ | CH₂CO₂Me | R | Me | H | S | H | H | 1 | | H | H | Me | H | - |
| I-81 | Me | CF₂Cl | R | Me | H | S | H | H | 1 | | H | H | Br | H | - |
| I-82 | Me | CF₂Cl | | H | H | | Me | Me | 1 | | H | Cl | F | H | O |
| I-83 | Me | CF₂Cl | R | H | H | | H | H | 1 | | F | H | H | H | O |
| I-84 | Me | CF₂Cl | R | H | H | | H | H | 1 | | H | H | H | H | O |
| I-85 | i-Pr | t-Bu | R | Me | H | S | H | H | 1 | | H | H | Me | H | - |
| I-86 | i-Pr | t-Bu | R | H | H | | H | H | 2 | | H | H | H | H | - |
| I-87 | Me | CF₃ | R | Me | H | S | H | H | 1 | | H | H | Me | H | - |
| I-88 | Et | CF₃ | R | Me | H | S | H | H | 1 | | H | H | Me | H | - |
| I-89 | Me | CF₃ | R | Me | H | S | H | H | 1 | | H | Me | Me | H | - |
| I-90 | Et | CF₃ | R | Me | H | S | H | H | 1 | | Me | H | Me | H | - |
| I-91 | Me | CF₂H | R | H | H | | H | H | 1 | | H | H | H | H | - |
| I-92 | Me | CF₂H | R | Me | H | | H | H | 1 | | H | H | H | H | - |
| I-93 | Me | CF₂H | R | H | H | | H | H | 1 | | Me | H | H | Me | - |
| I-94 | Me | CF₂H | | H | H | | H | H | 2 | | H | H | OMe | H | - |
| I-95 | Me | CF₂H | | H | H | | Me | Me | 1 | | H | Cl | F | H | O |
| I-96 | Me | CF₂H | | H | H | | Me | Me | 1 | | H | Cl | Cl | H | O |
| I-97 | Me | CF₂H | | H | H | | Me | Me | 1 | | H | H | F | H | O |
| I-98 | Me | CF₂H | | H | H | | Me | Me | 1 | | H | H | Me | H | O |
| I-99 | Me | CF₂H | | H | H | | Me | Me | 1 | | Me | H | H | H | O |
| I-100 | Me | CF₂H | | H | H | | H | H | 1 | | H | H | i-Pr | H | - |
| I-101 | Me | CF₂H | | H | H | | H | H | 1 | | H | H | t-Bu | H | - |
| I-102 | Me | CF₂H | | H | H | | H | H | 1 | | H | H | Et | H | - |
| I-103 | Me | CF₂H | | H | H | | H | H | 1 | | i-Pr | H | H | H | - |
| I-104 | Me | CF₂H | | H | H | | H | H | 1 | | H | H | I | H | - |
| I-105 | Me | CF₂H | | H | H | | H | H | 1 | | H | H | C≡C-Si(Me)₃ | H | - |
| I-106 | Me | CF₂H | | H | H | | H | H | 1 | | H | H | C≡CH | H | - |
| I-107 | Me | CF₂H | | H | H | | H | H | 1 | | H | H | C≡CC(Me)₃ | H | - |
| I-108 | Me | CF₂H | | H | H | | H | H | 1 | | H | H | C≡CCH₂OMe | H | - |
| I-109 | Me | CF₃ | | H | H | | H | H | 1 | | H | H | C≡CCH₂OMe | H | - |
| I-110 | Me | CF₂H | | H | H | | H | H | 1 | | H | H | C≡CMe | H | - |
| I-111 | Me | CF₃ | | H | H | | H | H | 1 | | H | H | I | H | - |
| I-112 | Me | CF₃ | | H | H | | H | H | 1 | | H | H | C≡CMe | H | - |
| I-113 | Et | CF₃ | | H | H | | H | H | 1 | | H | H | C≡CCH₂OMe | H | - |
| I-114 | Me | CF₃ | | H | H | | H | H | 1 | | H | H | C≡CH | H | - |
| I-115 | Me | CF₃ | | H | H | | H | H | 1 | | H | H | C≡CC(Me)₃ | H | - |
| I-116 | Me | CF₃ | | H | H | | H | H | 1 | | H | H | C≡C-Si(Me)₃ | H | - |
| I-117 | Et | CF₃ | | H | H | | H | H | 1 | | H | H | C≡CH | H | - |
| I-118 | Me | CF₂H | | H | H | | H | H | 1 | | H | H | CN | H | - |
| I-119 | Me | CF₂H | | H | H | | H | H | 1 | | H | H | CN | H | - |
| I-120 | Et | CF₂H | | H | H | | H | H | 1 | | H | H | CN | H | - |
| I-121 | t-Bu | CF₃ | | H | H | | H | H | 1 | | H | H | i-Pr | H | - |
| I-122 | Me | CF₂H | | H | H | | H | H | 2 | | H | H | OEt | H | - |
| I-123 | Me | CF₂H | | H | H | | H | H | 2 | | H | H | OCH₂CF₃ | H | - |
| I-124 | Me | CF₂H | | H | H | | H | H | 1 | | H | H | OCH₂CF₃ | H | O |
| I-125 | Me | CF₂H | | H | H | | H | H | 1 | | H | H | i-Pr | H | O |
| I-126 | Me | CF₂H | | H | H | | H | H | 1 | | Me | H | i-Pr | H | O |
| I-127 | Me | CF₂H | | H | H | | H | H | 1 | | Me | H | Me | H | O |
| I-128 | Me | CF₃ | | H | H | | H | H | 1 | | Me | H | Me | H | O |
| I-129 | Et | CF₃ | | H | H | | H | H | 1 | | Me | H | Me | H | O |
| I-130 | Me | CF₂H | | H | H | | Me | Me | 1 | | H | Me | H | H | O |
| I-131 | Me | CF₃ | | H | H | | Me | Me | 1 | | H | Me | H | H | O |
| I-132 | Me | CF₂Cl | | H | H | | Me | Me | 1 | | H | Me | H | H | O |
| I-133 | Me | CF₂H | | H | H | | Me | Me | 1 | | Me | Me | H | H | O |
| I-134 | Me | CF₂H | | H | H | | H | H | 1 | | H | H | Et | H | O |
| I-135 | Me | CF₃ | | H | H | | H | H | 1 | | H | H | Et | H | O |
| I-136 | Me | CF₂Cl | | H | H | | H | H | 1 | | H | H | Et | H | O |
| I-137 | Me | CF₂H | | Me | H | | H | H | 1 | | H | Cl | H | H | - |
| I-138 | Me | CF₃ | | Me | H | | H | H | 1 | | H | Cl | H | H | - |
| I-139 | Me | CF₂Cl | | Me | H | | H | H | 1 | | H | Cl | H | H | - |
| I-140 | Me | CF₂H | | Me | H | | H | H | 1 | | H | OMe | H | H | - |
| I-141 | Me | CF₂H | | Et | H | | H | H | 1 | | H | H | H | H | - |
| I-142 | Me | CF₂H | | Et | H | | H | H | 1 | | H | H | Me | H | - |
| I-143 | Me | CF₂H | | Et | H | | H | H | 1 | | H | H | Cl | H | - |
| I-144 | Me | CF₂H | | Me | H | | H | H | 1 | | H | H | F | H | - |
| I-145 | Me | CF₃ | | Me | H | | H | H | 1 | | H | H | F | H | - |
| I-146 | Me | CF₂H | | H | H | | H | H | 1 | | Me | H | H | H | - |
| I-147 | Me | CF₂Cl | | H | H | | H | H | 1 | | Me | H | H | H | - |
| I-148 | Me | CF₂H | | Me | H | | H | H | 1 | | Me | H | H | H | - |
| I-149 | Me | CF₃ | | Me | H | | H | H | 1 | | Me | H | H | H | - |
| I-150 | Me | CF₂Cl | | Me | H | | H | H | 1 | | Me | H | H | H | - |
| I-151 | Me | CF₂H | | H | H | | H | H | 1 | | H | Cl | H | H | - |
| I-152 | Me | CF₂H | | H | H | | H | H | 1 | | H | Cl | Cl | H | - |
| I-153 | Me | CF₃ | | H | H | | H | H | 2 | | H | Cl | Cl | H | - |
| I-154 | Me | CF₂H | | H | H | | H | H | 2 | | H | H | NO₂ | H | - |
| I-155 | Me | CF₃ | | H | H | | H | H | 2 | | H | H | NO₂ | H | - |
| I-156 | Me | CF₃ | | H | H | | H | H | 2 | | H | H | CN | H | - |
| I-157 | Me | CF₂H | | H | H | | H | H | 2 | | H | CN | H | H | - |
| I-158 | Me | CF₂H | | H | H | | H | H | 2 | | H | H | O-i-Pr | H | - |
| I-159 | Me | CF₂H | | H | H | | H | H | 2 | | H | OMe | H | H | - |
| I-160 | Me | Me | | H | H | | Me | Me | 1 | | H | Me | H | H | O |
| I-161 | Me | Et | | H | H | | Me | Me | 1 | | H | Me | H | H | O |
| I-162 | Me | CF₂H | | H | H | | H | H | 1 | | F | H | H | H | 0 |
| I-163 | Me | CF₃ | | H | H | | H | H | 1 | | F | H | H | H | O |
| I-164 | Me | Me | | H | H | | H | H | 1 | | F | H | H | H | O |
| I-165 | Me | Me | | H | H | | H | H | 1 | | H | H | i-Pr | H | O |
| I-166 | Me | CF₃ | | H | H | | H | H | 1 | | H | H | i-Pr | H | O |
| I-167 | Me | CF₂Cl | | H | H | | H | H | 1 | | H | H | i-Pr | H | O |
| I-168 | Me | Me | | H | H | | H | H | 1 | | Me | H | H | H | - |
| I-169 | Me | Et | | H | H | | H | H | 1 | | Me | H | H | H | - |
| I-170 | Me | Me | | H | H | | H | H | 2 | | F | H | H | H | - |
| I-171 | Me | CF₃ | | H | H | | H | H | 2 | | F | H | H | H | - |
| I-172 | Me | CF₂H | | H | H | | H | H | 2 | | F | H | H | H | - |
| I-173 | Me | CF₂H | | H | H | | H | H | 1 | | H | OMe | OMe | H | - |
| I-174 | Me | CF₃ | | H | H | | H | H | 1 | | H | OMe | OMe | H | - |
| I-175 | Me | CF₃ | | OMe | H | | H | H | 1 | | H | H | H | H | - |
| I-176 | Me | CF₂H | | OMe | H | | H | H | 1 | | H | H | H | H | - |
| I-177 | Me | CF₂H | R | H | H | | Me | Me | 1 | | H | H | H | H | O |
| I-178 | Me | CF₂H | R | H | H | | Me | Me | 1 | | H | H | H | H | CH₂ |
| I-179 | Me | Me | R | H | H | | Me | Me | 1 | | H | H | H | H | CH₂ |
| I-180 | Me | Me | R | H | H | | Me | Me | 1 | | H | H | H | H | - |
| I-181 | Me | Me | R | H | H | | H | H | 2 | | H | CF₂H | H | H | - |
| I-182 | Me | CF₃ | R | H | H | | H | H | 2 | | H | CF₂H | H | H | - |
| I-183 | Me | Me | R | H | H | | H | H | 2 | | CN | F | H | H | - |
| I-184 | Me | CF₃ | R | H | H | | H | H | 2 | | CN | F | H | H | - |
| I-185 | Me | CF₂H | R | H | H | | H | H | 2 | | CN | F | H | H | - |
| I-186 | Me | Me | R | H | H | | H | H | 2 | | Cl | F | H | H | - |
| I-187 | Me | CF₃ | R | H | H | | H | H | 2 | | Cl | F | H | H | - |
| I-188 | Me | Et | R | H | H | | H | H | 2 | | Cl | F | H | H | - |
| I-189 | Me | Et | R | H | H | | H | H | 2 | | F | H | OMe | H | - |
| I-190 | Me | CF₃ | R | H | H | | H | H | 2 | | F | H | OMe | H | - |
| I-191 | Me | CF₂H | R | H | H | | H | H | 2 | | F | H CH₂C | OMe | H | - |
| I-192 | Me | CF₂H | R | H | H | | H | H | 2 | | H | N CH₂C | OMe | H | - |
| I-193 | Me | CF₃ | R | H | H | | H | H | 2 | | H | N | OMe | H | - |
| I-194 | Me | CF₃ | R | H | H | | H | H | 2 | | H | Me | F | H | - |
| I-195 | Me | Me | R | H | H | | H | H | 2 | | H | Me | F | H | - |
| I-196 | Me | Me | R | H | H | | H | H | 2 | | H | CN | F | H | - |
| I-197 | Me | CF₂H | R | H | H | | H | H | 2 | | H | CN | F | H | - |
| I-198 | Me | Me | R | H | H | | H | H | 2 | | H | F | F | H | - |
| I-199 | Me | CF₃ | R | H | H | | H | H | 2 | | H | F | F | H | - |
| I-200 | Me | CF₃ | R | H | H | | H | H | 2 | | H | t-Bu | H | H | - |
| I-201 | Me | CF₂H | R | H | H | | H | H | 2 | | H | H | N(Me)₂ | H | - |
| I-202 | Me | Me | R | H | H | | H | H | 1 | | OMe | H | F | H | - |
| I-203 | Me | CF₃ | R | H | H | | H | H | 1 | | OMe | H | F | H | - |
| I-204 | Me | CF₃ | R | H | H | | H | H | 1 | | OMe | Me | F | H | - |
| I-205 | Me | CF₃ | R | H | H | | H | H | 1 | | H | Me | F | H | - |
| I-206 | Me | CF₃ | R | H | H | | H | H | 1 | | Cl | Me | H | H | - |
| I-207 | Me | CF₂H | R | H | H | | H | H | 1 | | Cl | Me | H | H | - |
| I-208 | Me | Me | R | H | H | | H | H | 1 | | F | H | CN | H | O |
| I-209 | Me | CF₃ | R | H | H | | H | H | 1 | | F | H | CN | H | O |
| I-210 | Me | CF₂Cl | R | H | H | | H | H | 1 | | F | H | CN | H | O |
| I-211 | Me | CF₂Cl | R | H | H | | H | H | 1 | | F | H | Br | H | O |
| I-212 | Me | CF₂H | R | H | H | | H | H | 1 | | F | H | Br | H | O |
| I-213 | Me | CF₂H | R | H | H | | H | H | 1 | | F | H | C≡CH | H | O |
| I-214 | Me | Me | | H | H | | H | H | 1 | | Cl | H | Br | H | O |
| I-215 | Me | CF₃ | | H | H | | H | H | 1 | | Cl | H | Br | H | O |
| I-216 | Me | CF₃ | | H | H | | H | H | 1 | | Cl | H | Br | H | O |
| I-217 | Me | Me | | H | H | | | | 0 | | H | H | Me | H | O |
| I-218 | Me | CF₃ | | H | H | | | | 0 | | H | H | Me | H | O |
| I-219 | Me | CF₂Cl | | H | H | | | | 0 | | H | H | Me | H | O |
| I-220 | Me | CF₂H | | H | H | | | | 0 | | H | H | Me | H | O |
| I-221 | Me | Me | R | H | H | | H | H | 1 | | H | H | CH₂CN | H | O |
| I-222 | Me | CF₃ | R | H | H | | H | H | 1 | | H | H | CH₂CN | H | O |
| I-223 | Me | CF₂H | R | H | H | | H | H | 1 | | H | H | CH₂CN | H | O |
| I-224 | Me | Me | R | H | H | | H | H | 1 | | H | H | (CH₂)₂CN | H | O |
| I-225 | Me | Me | R | H | H | | H | H | 1 | | H | H | (CH₂)₂CN | H | O |
| I-226 | Me | CF₂H | R | H | H | | H | H | 1 | | H | H | (CH₂)₂CN | H | O |
| I-227 | Me | CF₂H | R | H | H | | H | H | 1 | | H | H | CH₂OMe | H | O |
| I-228 | Me | CF₂H | R | H | H | | H | H | 1 | | H | H | CH₂OMe | H | - |
| I-229 | Me | CF₂H | R | H | H | | H | H | 1 | | CN | H | H | H | O |
| I-230 | Me | Me | R | H | H | | H | H | 1 | | CN | H | H | H | O |
| I-231 | Me | Me | | H | H | | H | H | 1 | | H | H | Me | H | O |
| I-232 | Me | CF₃ | | H | H | | H | H | 1 | | H | H | Me | H | O |
| I-233 | Me | CF₂H | | H | H | | H | H | 1 | | H | H | Me | H | O |
| I-234 | Me | CF₂H | | H | H | | H | H | 1 | | Br | H | Me | H | O |
| I-235 | Me | CF₂H | R | Me | H | S | H | H | 1 | | H | H | Br | H | - |
| I-236 | Me | CF₃ | R | H | H | | H | H | 1 | | H | H | H | H | - |
| I-237 | Me | CF₃ | | H | H | | H | H | 1 | | H | H | OMe | H | O |
| I-238 | Me | Me | | H | H | | H | H | 1 | | H | H | Et | H | O |
| I-239 | Me | Me | | Me | Me | | H | H | 2 | | H | H | H | H | - |
| I-240 | Me | Me | | H | H | | Me | H | 1 | rac | H | H | H | H | CH₂ |
| I-241 | Me | CF₂H | | H | H | | H | H | 1 | | H | H | H | H | CHMe |
| I-242 | Me | CF₂Cl | | H | H | | H | H | 1 | | H | H | H | H | CHMe |
| I-243 | Me | CF₃ | | H | H | | H | H | 1 | | H | H | H | H | CHMe |
| I-244 | Me | Me | | H | H | | H | H | 1 | | H | H | H | H | CHMe |
| I-245 | Me | Et | | H | H | | H | H | 1 | | H | H | H | H | CHMe |
| I-246 | Me | i-Pr | | H | H | | H | H | 1 | | H | H | H | H | CHMe |
| I-247 | Me | c-Pr | | H | H | | H | H | 1 | | H | H | H | H | CHMe |
| I-248 | Me | CH₃ | | H | H | | H | H | 1 | | OMe | H | H | H | NMe |
| I-249 | Me | CF₃ | | H | H | | H | H | 1 | | OMe | H | H | H | NMe |
| I-250 | Me | CF₂H | | H | H | | H | H | 1 | | OMe | H | H | H | NMe |
| I-251 | Me | Me | | H | H | | H | H | 1 | | OMe | H | H | H | NEt |
| I-252 | Me | CF₃ | | H | H | | H | H | 1 | | OMe | H | H | H | NEt |
| I-253 | Me | CF₂H | | H | H | | H | H | 1 | | OMe | H | H | H | NEt |
| I-254 | Me | Me | | H | H | | H | H | 1 | | H | H | OMe | H | NMe |
| I-255 | Me | CF₃ | | H | H | | H | H | 1 | | H | H | OMe | H | NMe |
| I-256 | Me | CF₂H | | H | H | | H | H | 1 | | H | H | OMe | H | NMe |
| I-257 | Me | Me | | H | H | | H | H | 1 | | H | H | OMe | H | NEt |
| I-258 | Me | CF₃ | | H | H | | H | H | 1 | | H | H | OMe | H | NEt |
| I-259 | Me | CF₂H | | H | H | | H | H | 1 | | H | H | OMe | H | NEt |

Ein weiterer Gegenstand der vorliegenden Erfindung sind Verfahren zur Herstellung entsprechender Verbindungen der allgemeinen Formel (I) und/oder deren Salze und/oder deren agrochemisch verträglichen quaternierten Stickstoff-Derivate:
a.) Zur Herstellung von Verbindungen der allgemeinen Formel (I) 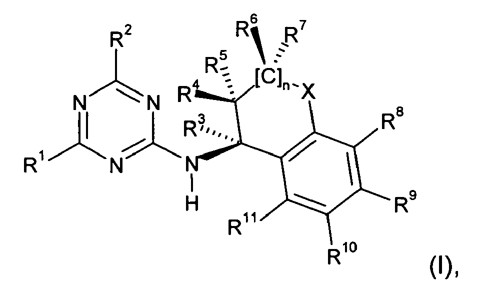 in welchen die Reste R¹ bis R¹¹, n und X die vorstehenden Bedeutungen aufweisen, kann man eine Verbindung der allgemeinen Formel (II) 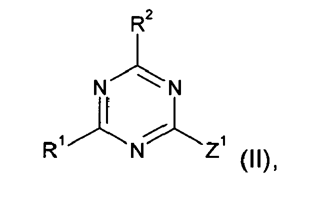 worin R¹ und R² die vorstehende Bedeutung aufweisen und Z¹ einen austauschfähigen Rest oder eine Abgangsgruppe, wie insbesondere Chlor, Trichlormethyl, (C₁-C₄)-Alkylsulfanyl, (C₁-C₄)-Alkylsulfonyl, unsubstituiertes oder substituiertes Phenyl-(C₁-C₄)-alkylsulfonyl oder (C₁-C₄)-Alkylphenylsulfonyl, bedeutet, mit einem Amin der allgemeinen Formel (III) oder einem Säureadditionssalz davon 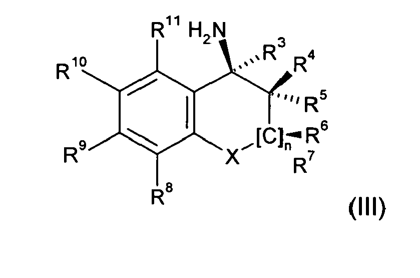 umgesetzt wird, wobei die Reste R³ bis R¹¹, n und X die vorstehende Bedeutung aufweisen.

Entsprechende Verfahren sind prinzipiell bekannt (z.B. Z¹ = S-Me: G. Seitz, R. John, Chem. Ber. 1989, 122(7), 1381; Z¹ = S-Alkyl, Oxidatation zu -SO₂Alkyl: T. Masquelin, Y. Delgado, V. Baumle Tetrahedron Lett. 1998, 39(32), 5725-5726; Z¹ = Cl: Takeda Chem. Ind. Ltd.: JP 217766, 1962; Chem Abstr.: 64: 14200g, 1966; Z¹ = CCl₃: N. Kuboyma et al. J. Pesticide Sci. 1998, 23, 268 - 274). Die Anwendung der Verfahren scheitert jedoch häufig, da entweder die Reaktivität der Amine der allgemeinen Formel (III) oder deren Säureadditionssalze nicht ausreicht oder bei den angewendeten Verfahren sich die Produkte zersetzen.

Meist sind bei der Reaktion deutlich höhere Temperaturen als bei offenkettigen Derivaten erforderlich. Vorteilhaft ist die Verwendung eines geschlossenen Reaktionstubes anstelle eines offenen Reaktionsgefäßes und die Verwendung von eines Mikrowellen-Reaktor zur Erhitzung der jeweiligen Reaktionsmischung. Die Reaktionen können durchgeführt werden unter Zugabe einer äquimolaren oder bis fünffach überschüssigen Menge einer geeigneten anorganischen Base wie Kaliumcarbonat oder einer organischen Base wie Triethylamine oder Dicyclohexylethylamine als äquimolare Mischungen oder unter Verwendung eines Überschusses an Amin der allgemeinen Formel (III) oder deren Säureadditionssalze, wobei die Salze in Situ durch die Zugabe einer geeigneten anorganischen Base wie Kaliumcarbonat oder einer organischen Base wie Triethylamine oder Dicyclohexylethylamine freigesetzt werden, in einen geeigneten protischen Lösemittel wie Dimethylformamid, Dimethylacetamid oder bevorzugt 1-Methyl-2-pyrrolidon bei einer Reaktionstemperatur von 50 bis 180°C und einer Reaktionsdauer von 20 Minuten bis 10 Stunden. Die Isolierung der Reaktionsprodukte kann chromatographisch direkt aus der Reaktionsmischung oder nach wässriger Aufarbeitung erfolgen.

Die Verbindungen der allgemeinen Formel (II) sind entweder kommerziell verfügbar oder können entsprechend bekannten Vorschriften hergestellt werden. z.B. Z¹ = Cl: Schroeder, Grundmann, JACS, 78, 1956, 2447; Z¹ = S-Me: F.C. Schäfer, J. Org. Chem. 1962, 27, 3608; Z¹ = CCl₃: F.C. Schäfer J. Org. Chem. 1964, 29, 1527; C. Grundmann, Chem. Ber. 1964, 97, 3262; K. Wakabayashi et al. Bull. Chem Soc. Jpn. 1969, 42, 2924 und 2931; K. Koizumi et al., Pesticide Science, 1999, 55(6), 642.

Die Amine der allgemeinen Formel (III) oder die Säureadditionssalz davon sind kommerziell verfügbar oder deren Synthese ist in WO 2004/069814 A1 beschrieben.
b.) Zur Herstellung der Verbindungen der Struktur I können auch bereits der Formel entsprechende Derivate modifiziert werden. Beispielsweise können halogenierte Alkylgruppen hydriert werden, entsprechende Verfahren sind z.B. von V.I. Kalarev et. al. Khim Geterotsikl Soedin 1995 (5), 667-673 und H.L. Nyquist, B. Wolfe, J. Org. Chem. 1974, 39, 2591 publiziert.
   Gegebenenfalls können auch Derivate mit chemisch geeigneten Resten R⁸ - R¹¹ in andere Derivate überführt werden, beispielsweise können Halogene übergangsmetallkatalysiert in entsprechende Alkene oder Alkine überführt werden.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, lowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasen- unterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und A. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Weiterer Gegenstand der Erfindung ist aufgrund der herbiziden Eigenschaft der Verbindungen der allgemeinen Formel (I) auch die Verwendung der erfindungsgemäßen Verbindungen der allgemeinen Formel (I) als Herbizide zur Bekämpfung von Schadpflanzen.

Herbizide werden in landwirtschaftlich genutzten Kulturen während verschiedener Anbauphasen eingesetzt. So erfolgt die Applikation einiger Produkte schon vor oder während der Saat. Andere werden ausgebracht, bevor die Kulturpflanze aufläuft, d.h. bevor der Keimling die Erdoberfläche durchbricht (Vorauflauf-Herbizide). Nachauflauf-Herbizide schließlich werden verwendet, wenn von der Kulturpflanze entweder bereits die Keimblätter oder Laubblätter ausgebildet sind.

Die erfindungsgemäßen Verbindungen können dabei sowohl im Vorlauf als auch im Nachlauf angewendet werden, wobei eine Verwendung der erfindungsgemäßen Verbindungen im Vorlauf bevorzugt ist.

Die Vorauflauf-Behandlung schließt sowohl die Behandlung der Anbaufläche vor der Aussaat (ppi = pre plant incorporation) als auch die Behandlung der angesäten, aber noch nicht bewachsenen Anbauflächen ein.

Die erfindungsgemäßen Verbindungen der Formel (I) und deren Salze, im folgenden auch synonym zusammen auch als Verbindungen der Formel (I) bezeichnet, weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Im einzelnem seien beispielhaft einige Vertreter der mono- und dikotylen Unkrautflora genannt, die durch die erfindungsgemäßen Verbindungen der allgemeinen Formel (I) kontrolliert werden können, ohne daß durch die Nennung eine Beschränkung auf bestimmte Arten erfolgen soll.

Auf der Seite der monokotylen Unkrautarten werden z. B. Agrostis, Alopecurus, Apera, Avena, Brachicaria, Bromus, Dactyloctenium, Digitaria, Echinochloa, Eleocharis, Eleusine, Festuca, Fimbristylis, Ischaemum, Lolium, Monochoria, Panicum, Paspalum, Phalaris, Phleum, Poa, Sagittaria, Scirpus, Setaria, Sphenoclea, sowie Cyperusarten vorwiegend aus der annuellen Gruppe und auf Seiten der perennierenden Spezies Agropyron, Cynodon, Imperata sowie Sorghum und auch ausdauernde Cyperusarten gut erfaßt.

Bei dikotylen Unkrautarten erstreckt sich das Wirkungsspektrum auf Arten wie z. B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern. Außerdem wird herbizide Wirkung bei dikotylen Unkräutern wie Ambrosia, Anthemis, Carduus, Centaurea, Chenopodium, Cirsium, Convolvulus, Datura, Emex, Galeopsis, Galinsoga, Lepidium, Lindernia, Papaver, Portlaca, Polygonum, Ranunculus, Rorippa, Rotala, Seneceio, Sesbania, Solanum, Sonchus, Taraxacum, Trifolium, Urtica und Xanthium beobachtet.

Werden die erfindungsgemäßen Verbindungen der allgemeinen Formel (I) vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe der allgemeinen Formel (I) auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen der allgemeinen Formel (I) eine ausgezeichnete herbizide Aktivität gegenüber mono- und dikotylen Unkräutern aufweisen, werden Kulturpflanzen wirtschaftlich bedeutender Kulturen wie z. B. Weizen, Gerste, Roggen, Reis, Mais, Zuckerrübe, Baumwolle, Raps und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüber hinaus weisen die erfindungsgemäßen Substanzen der allgemeinen Formel (I) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z. B. durch Auslösen von Desikkation und Wuchsstauchung eingesetzt werden. Desweiteren eignen sie sich auch zur generellen Steuerung und Hemmung von unerwünschtem vegetativem Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z. B. Hitze, Kälte, Trockenheit, Salz und ultraviolette Strahlung liegen.

Bevorzugt ist die Anwendung der erfindungsgemäßen Verbindungen der allgemeinen Formel (I) oder deren Salze in wirtschaftlich bedeutenden transgenen Kulturen von Nutz- und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der allgemeinen Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP 0221044, EP 0131624). Beschrieben wurden beispielsweise in mehreren Fällen
- gentechnische Veränderungen von Kulturpflanzen zwecks Modifikation der in den Pflanzen synthetisierten Stärke (z. B. WO 92/011376, WO 92/014827, WO 91/019806),
- transgene Kulturpflanzen, welche gegen bestimmte Herbizide vom Typ Glufosinate (vgl. z. B. EP 0242236, EP 0242246) oder Glyphosate (WO 92/000377) oder der Sulfonylharnstoffe (EP 0257993, US 5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP 0142924, EP 0193259),
- transgene Kulturpflanzen mit modifizierter Fettsäurezusammensetzung (WO 91/013972),
- gentechnisch veränderte Kulturpflanzen mit neuen Inhalts- oder Sekundärstoffen z. B. neuen Phytoalexinen, die eine erhöhte Krankheitsresistenz verursachen (EP 0309862, EP 0464461),
- gentechnisch veränderte Pflanzen mit reduzierter Photorespiration, die höhere Erträge und höhere Stresstoleranz aufweisen (EP 0305398),
- transgene Kulturpflanzen, die pharmazeutisch oder diagnostisch wichtige Proteine produzieren ("molecular pharming"),
- transgene Kulturpflanzen, die sich durch höhere Erträge oder bessere Qualitat auszeichnen,
- transgene Kulturpflanzen die sich durch eine Kombinationen z. B. der o. g. neuen Eigenschaften auszeichnen ("gene stacking").

Zahlreiche molekularbiologische Techniken, mit denen neue transgene Pflanzen mit veränderten Eigenschaften hergestellt werden können, sind im Prinzip bekannt; siehe z. B. I. Potrykus und G. Spangenberg (eds.) Gene Transfer to Plants, Springer Lab Manual (1995), Springer Verlag Berlin, Heidelberg oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen der allgemeinen Formel (I) in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe der allgemeinen Formel (I) in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der allgemeinen Formel (I) als Herbizide zur Bekämpfung von Schadpflanzen in transgenen Kulturpflanzen.

Die Verbindungen der allgemeinen Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986; Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösemittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z. B. Insektiziden, Akariziden, Herbiziden, Fungiziden, sowie mit Safenern, Düngemitteln und/oder Wachstumsregulatoren herstellen, z. B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z. B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-Dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösemittel z. B. Butanol, Cyclohexanon, Dimethylformamid, Xylol oder auch höhersiedenden Aromaten oder Kohlenwasserstoffen oder Mischungen der organischen Lösemittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Cadodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z. B. Sorbitanfettsäureester oder Polyoxethylensorbitanester wie z. B. Polyoxyethylensorbitanfettsäureester.

Stäubemittel erhält man durch Vermahlen des Wirkstoffes mit fein verteilten festen Stoffen, z. B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z. B. oben bei den anderen Formulierungstypen bereits aufgeführt sind, hergestellt werden.

Emulsionen, z. B. Öl-in-Wasser-Emulsionen (EW), lassen sich beispielsweise mittels Rühren, Kolloidmühlen und/oder statischen Mischern unter Verwendung von wäßrigen organischen Lösemitteln und gegebenenfalls Tensiden, wie sie z. B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z. B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt. Zur Herstellung von Teller-, Fließbett-, Extruder- und Sprühgranulate siehe z. B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z. B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I).

In Spritzpulvern beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe u.a. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösemittel, Füll-, Träger- und Farbstoffe, Entschäumer, Verdunstungshemmer und den pH-Wert und die Viskosität beeinflussende Mittel.

Die Verbindungen der allgemeinen Formel (I) oder deren Salze können als solche oder in Form ihrer Zubereitungen (Formulierungen) mit anderen pestizid wirksamen Stoffen, wie z. B. Insektiziden, Akariziden, Nematiziden, Herbiziden, Fungiziden, Safenern, Düngemitteln und/oder Wachstumsregulatoren kombiniert eingesetzt werden, z. B. als Fertigformulierung oder als Tankmischungen.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 13th edition, The British Crop Protection Council and the Royal Soc. of Chemistry, 2003 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:

Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminopyralid, Amitrole, Ammoniumsulfamat, Ancymidol, Anilofos, Asulam, Atrazine, Azafenidin, Azimsulfuron, Aziprotryn, BAH-043, BAS-140H, BAS-693H, BAS-714H, BAS-762H, BAS-776H, BAS-800H, Beflubutamid, Benazolin, Benazolin-ethyl, Bencarbazone, Benfluralin, Benfuresate, Bensulide, Bensulfuron-methyl, Bentazone, Benzfendizone, Benzobicyclon, Benzofenap, Benzofluor, Benzoylprop, Bifenox, Bilanafos, Bilanafosnatrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenac-natrium, Chlorfenprop, Chlorflurenol, Chlorflurenol-methyl, Chloridazon, Chlorimuron, Chlorimuron-ethyl, Chlormequat-chlorid, Chlornitrofen, Chlorophthalim, Chlorthaldimethyl, Chlorotoluron, Chlorsulfuron, Cinidon, Cinidon-ethyl, Cinmethylin, Cinosulfuron, Clethodim, Clodinafop Clodinafop-propargyl, Clofencet, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyr-natrium, Dimefuron, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuronmethyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3fluorpropyl)-4,5-dihydro-5-oxo-1H-tetrazol-1-yl]-phenyl]-ethansulfonamid, Fenoprop, Fenoxaprop, Fenoxaprop-P, Fenoxaprop-ethyl, Fenoxaprop-P-ethyl, Fentrazamide, Fenuron, Flamprop, Flamprop-M-isopropyl, Flamprop-M-methyl, Flazasulfuron, Florasulam, Fluazifop, Fluazifop-P, Fluazifop-butyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumiclorac-pentyl, Flumioxazin, Flumipropyn, Fluometuron, Fluorodifen, Fluoroglycofen, Fluoroglycofenethyl, Flupoxam, Flupropacil, Flupropanate, Flupyrsulfuron, Flupyrsulfuron-methylsodium, Flurenol, Flurenol-butyl, Fluridone, Flurochloridone, Fluroxypyr, Fluroxypyrmeptyl, Flurprimidol, Flurtamone, Fluthiacet, Fluthiacet-methyl, Fluthiamide, Fomesafen, Foramsulfuron, Forchlorfenuron, Fosamine, Furyloxyfen, Gibberellinsäure, Glufosinate, L-Glufosinate, L-Glufosinate-ammonium, Glufosinate-ammonium, Glyphosate, Glyphosate-isopropylammonium, H-9201, Halosafen, Halosulfuron, Halosulfuron-methyl, Haloxyfop, Haloxyfop-P, Haloxyfop-ethoxyethyl, Haloxyfop-P-ethoxyethyl, Haloxyfop-methyl, Haloxyfop-P-methyl, Hexazinone, HNPC-9908, HOK-201, HW-02, Imazamethabenz, Imazamethabenz-methyl, Imazamox, Imazapic, Imazapyr, Imazaquin, Imazethapyr, Imazosulfuron, Inabenfide, Indanofan, Indolessigsäure (IAA), 4-Indol-3-ylbuttersäure (IBA), lodosulfuron, Iodosulfuron-methyl-natrium, loxynil, Isocarbamid, Isopropalin, Isoproturon, Isouron, Isoxaben, Isoxachlortole, Isoxaflutole, Isoxapyrifop, IDH-100, KUH-043, KUH-071, Karbutilate, Ketospiradox, Lactofen, Lenacil, Linuron, Maleinsäurehydrazid, MCPA, MCPB, MCPB-methyl, -ethyl und -natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuron-methyl, Mesotrione, Methabenzthiazuron, Metam, Metamifop, Metamitron, Metazachlor, Methazole, Methoxyphenone, Methyldymron, 1-Methylcyclopropen, Methylisothiocyanat, Metobenzuron, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamide-dihydrogensulfat, Monolinuron, Monosulfuron, Monuron, MT 128, MT-5950, d. h. N-[3-Chlor-4-(1-methylethyl)-phenyl]-2-methylpentanamid, NGGC-011, Naproanilide, Napropamide, Naptalam, NC-310, d.h. 4-(2,4-dichlorobenzoyl)-1-methyl-5-benzyloxypyrazole, Neburon, Nicosulfuron, Nipyraclofen, Nitralin, Nitrofen, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquatdichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadionecalcium, Prohydrojasmone, Prometon, Prometryn, Propachlor, Propanil, Propaquizafop, Propazine, Propham, Propisochlor, Propoxycarbazone, Propoxycarbazonenatrium, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron-ethyl, Pyrazoxyfen, Pyribambenz, Pyribambenz-isopropyl, Pyribenzoxim, Pyributicarb, Pyridafol, Pyridate, Pyriftalid, Pyriminobac, Pyriminobac-methyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Secbumeton, Sethoxydim, Siduron, Simazine, Simetryn, SN-106279, Sulcotrione, Sulfallate (CDEC), Sulfentrazone, Sulfometuron, Sulfometuron-methyl, Sulfosate (Glyphosate-trimesium), Sulfosulfuron, SYN-523, SYP-249, SYP-298, SYP-300, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, TH-547, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triallate, Triasulfuron, Triaziflam, Triazofenamide, Tribenuron, Tribenuron-methyl, Trichloressigsäure (TCA), Triclopyr, Tridiphane, Trietazine, Trifloxysulfuron, Trifloxysulfuronnatrium, Trifluralin, Triflusulfuron, Triflusulfuron-methyl, Trimeturon, Trinexapac, Trinexapac-ethyl, Tritosulfuron, Tsitodef, Uniconazole, Uniconazole-P, Vernolate, ZJ-0166, ZJ-0270, ZJ-0543, ZJ-0862 sowie die folgenden Verbindungen

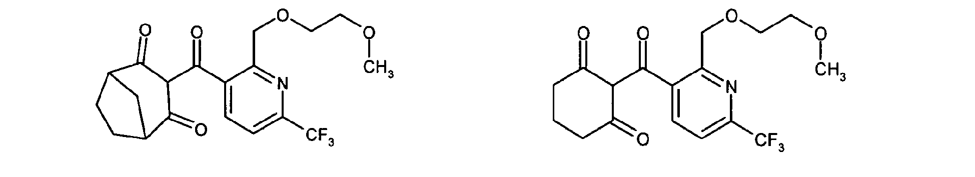

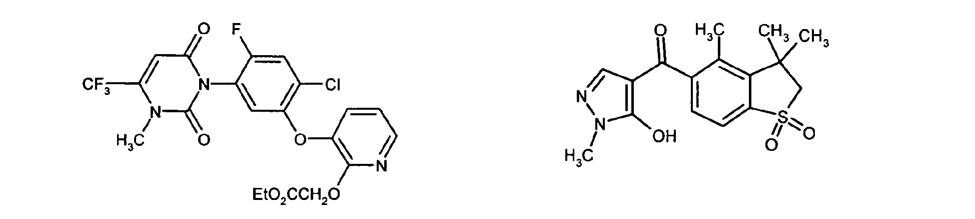

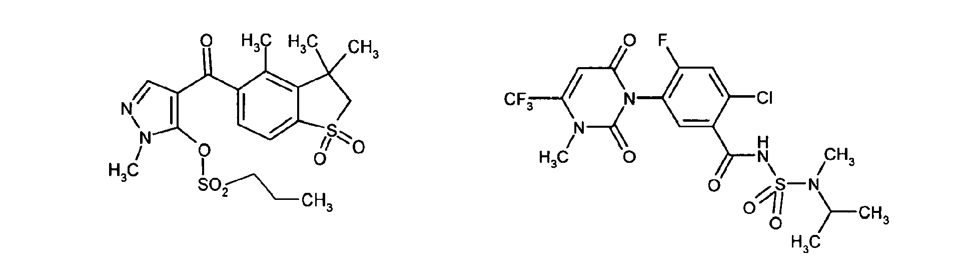

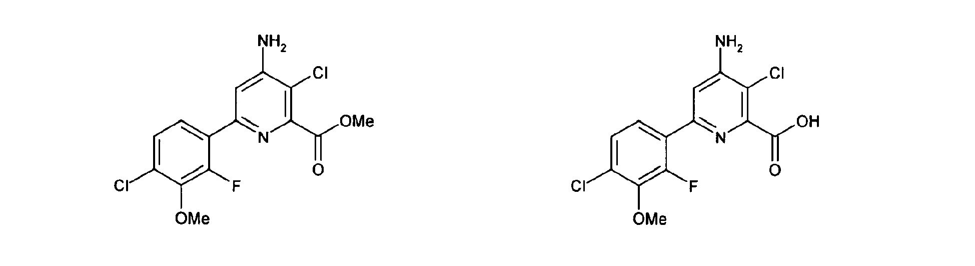

Von besonderem Interesse ist die selektive Bekämpfung von Schadpflanzen in Kulturen von Nutz- und Zierpflanzen. Obgleich die erfindungsgemäßen Verbindungen der allgemeinen Formel (I) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen erfindungsgemäßer Verbindungen der allgemeinen Formel (I) von besonderem Interesse, welche die Verbindungen der allgemeinen Formel (I) bzw. deren Kombinationen mit anderen Herbiziden oder Pestiziden und Safenern enthalten. Die Safener, welche in einem antidotisch wirksamen Gehalt eingesetzt werden, reduzieren die phytotoxischen Nebenwirkungen der eingesetzten Herbizide/Pestizide, z. B. in wirtschaftlich bedeutenden Kulturen wie Getreide (Weizen, Gerste, Roggen, Mais, Reis, Hirse), Zuckerrübe, Zuckerrohr, Raps, Baumwolle und Soja, vorzugswiese Getreide. Folgende Gruppen von Verbindungen kommen beispielsweise als Safener für die Verbindungen (I) alleinig oder aber in deren Kombinationen mit weiteren Pestiziden in Frage:

Die Safener sind vorzugsweise ausgewählt aus der Gruppe bestehend aus:
S1) Verbindungen der Formel (S1), 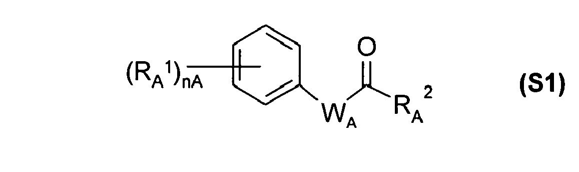 wobei die Symbole und Indizes folgende Bedeutungen haben:
   - n_{A}: ist eine natürliche Zahl von 0 bis 5, vorzugsweise 0 bis 3;
   - R_{A}¹: ist Halogen, (C₁-C₄)Alkyl, (C₁-C₄)Alkoxy, Nitro oder (C₁-C₄)Haloalkyl;
   - W_{A}: ist ein unsubstituierter oder substituierter divalenter heterocyclischer Rest aus der Gruppe der teilungesättigten oder aromatischen Fünfring-Heterocyclen mit 1 bis 3 Heteroringatomen aus der Gruppe N und O, wobei mindestens ein N- Atom und höchstens ein O-Atom im Ring enthalten ist, vorzugsweise ein Rest 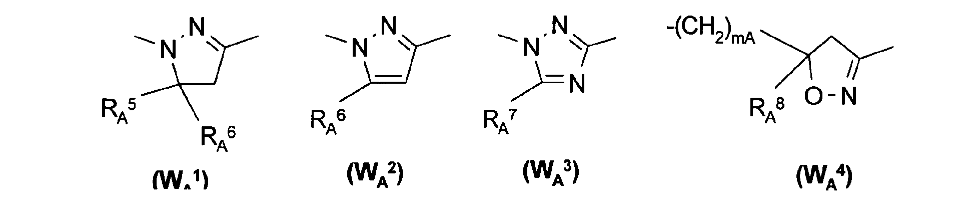 aus der Gruppe (W_{A}¹) bis (W_{A}⁴),
   - m_{A}: ist 0 oder 1;
   - R_{A}²: ist OR_{A}³, SR_{A}³ oder NR_{A}³R_{A}⁴ oder ein gesättigter oder ungesättigter 3- bis 7- gliedriger Heterocyclus mit mindestens einem N-Atom und bis zu 3 Hetero- atomen, vorzugsweise aus der Gruppe O und S, der über das N-Atom mit der Carbonylgruppe in (S1) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{A}³, NHR_{A}⁴ oder N(CH₃)₂, insbesondere der Formel OR_{A}³;
   - R_{A}³: ist Wasserstoff oder ein unsubstituierter oder substituierter aliphatischer Koh- lenwasserstoffrest, vorzugsweise mit insgesamt 1 bis 18 C-Atomen;
   - R_{A}⁴: ist Wasserstoff, (C₁-C₆)Alkyl, (C₁-C₆)Alkoxy oder substituiertes oder unsubsti- tuiertes Phenyl;
   - R_{A}⁵: ist H, (C₁-C₈)Alkyl, (C₁-C₈)Haloalkyl, (C₁-C₄)Alkoxy(C₁-C₈)Alkyl, Cyano oder COOR_{A}⁹, worin R_{A}⁹ Wasserstoff, (C₁-C₈)Alkyl, (C₁-C₈)Haloalkyl, (C₁-C₄)Alkoxy- (C₁-C₄)alkyl, (C₁-C₆)Hydroxyalkyl, (C₃-C₁₂)Cycloalkyl oder Tri-(C₁-C₄)-alkyl-silyl ist;
   - R_{A}⁶, R_{A}⁷, R_{A}⁸: sind gleich oder verschieden Wasserstoff, (C₁-C₈)Alkyl, (C₁-C₈)Halo- alkyl, (C₃-C₁₂)Cycloalkyl oder substituiertes oder unsubstituiertes Phenyl;
   vorzugsweise:
   a) Verbindungen vom Typ der Dichlorphenylpyrazolin-3-carbonsäure (S1^{a}), vorzugsweise Verbindungen wie 1-(2,4-Dichlorphenyl)-5-(ethoxycarbonyl)-5-methyl-2-pyrazolin-3-carbonsäure, 1-(2,4-Dichlorphenyl)-5-(ethoxycarbonyl)-5-methyl-2-pyrazolin-3-carbonsäure-ethylester (S1-1) ("Mefenpyr-diethyl"), und verwandte Verbindungen, wie sie in der WO-A-91/07874 beschrieben sind;
   b) Derivate der Dichlorphenylpyrazolcarbonsäure (S1^{b}), vorzugsweise Verbindungen wie 1-(2,4-Dichlorphenyl)-5-methyl-pyrazol-3-carbonsäureethyl-ester (S1-2), 1-(2,4-Dichlorphenyl)-5-isopropyl-pyrazol-3-carbonsäureethyl-ester (S1-3), 1-(2,4-Dichlorphenyl)-5-(1,1-dimethyl-ethyl)pyrazol-3-carbon-säureethyl-ester (S1-4) und verwandte Verbindungen, wie sie in EP-A-333 131 und EP-A-269 806 beschrieben sind;
   c) Derivate der 1,5-Diphenylpyrazol-3-carbonsäure (S1^{C}), vorzugsweise Verbindungen wie 1-(2,4-Dichlorphenyl)-5-phenylpyrazol-3-carbonsäureethylester (S1 -5), 1-(2-Chlorphenyl)-5-phenylpyrazol-3-carbonsäuremethylester (S1-6) und verwandte Verbindungen wie sie beispielsweise in der EP-A-268554 beschrieben sind;
   d) Verbindungen vom Typ der Triazolcarbonsäuren (S1^{d}), vorzugsweise Verbindungen wie Fenchlorazol(-ethylester), d.h. 1-(2,4-Dichlorphenyl)-5-trichlormethyl-(1H)-1,2,4-triazol-3-carbonsäureethylester (S1-7), und verwandte Verbindungen wie sie in EP-A-174 562 und EP-A-346 620 beschrieben sind;
   e) Verbindungen vom Typ der 5-Benzyl- oder 5-Phenyl-2-isoxazolin-3- carbonsäure oder der 5,5-Diphenyl-2-isoxazolin-3-carbonsäure (S1^{e}), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-carbonsäure (S1-10) oder 5,5-Diphenyl-2-isoxazolin-carbonsäureethylester (S1-11) ("Isoxadifen-ethyl") oder -n-propylester (S1-12) oder der 5-(4-Fluorphenyl)-5-phenyl-2-isoxazolin-3-carbonsäureethylester (S1-13), wie sie in der Patentanmeldung WO-A-95/07897 beschrieben sind.
S2) Chinolinderivate der Formel (S2), 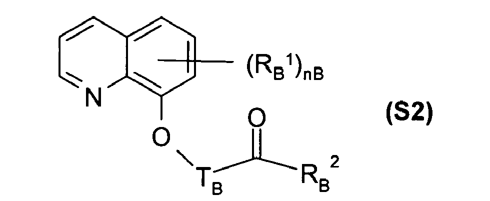 wobei die Symbole und Indizes folgende Bedeutungen haben:
   - R_{B}¹: ist Halogen, (C₁-C₄)Alkyl, (C₁-C₄)Alkoxy, Nitro oder (C₁-C₄)Haloalkyl;
   - n_{B}: ist eine natürliche Zahl von 0 bis 5, vorzugsweise 0 bis 3;
   - R_{B}²: ist OR_{B}³ SR_{B}³ oder NR_{B}³R_{B}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S2) verbunden ist und un- substituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{B}³, NHR_{B}⁴ oder N(CH₃)₂, insbesondere der Formel OR_{B}³;
   - R_{B}³: ist Wasserstoff oder ein unsubstituierter oder substituierter aliphatischer Koh- lenwasserstoffrest, vorzugsweise mit insgesamt 1 bis 18 C-Atomen;
   - R_{B}⁴: ist Wasserstoff, (C₁-C₆)Alkyl, (C₁-C₆)Alkoxy oder substituiertes oder unsubsti- tuiertes Phenyl;
   - T_{B}: ist eine (C₁ oder C₂)-Alkandiylkette, die unsubstituiert oder mit einem oder zwei (C₁-C₄)Alkylresten oder mit [(C₁-C₃)-Alkoxy]-carbonyl substituiert ist;
   vorzugsweise:
   a) Verbindungen vom Typ der 8-Chinolinoxyessigsäure (S2^{a}), vorzugsweise
      (5-Chlor-8-chinolinoxy)essigsäure-(1-methylhexyl)ester ("Cloquintocet-mexyl") (S2-1),
      (5-Chlor-8-chinolinoxy)essigsäure-(1,3-dimethyl-but-1-yl)ester (S2-2),
      (5-Chlor-8-chinolinoxy)essigsäure-4-allyloxy-butylester (S2-3),
      (5-Chlor-8-chinolinoxy)essigsäure-1-allyloxy-prop-2-ylester (S2-4),
      (5-Chlor-8-chinolinoxy)essigsäureethylester (S2-5),
      (5-Chlor-8-chinolinoxy)essigsäuremethylester (S2-6),
      (5-Chlor-8-chinolinoxy)essigsäureallylester (S2-7),
      (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie (5-Chlor-8-chinolinoxy)essigsäure (S2-10), deren Hydrate und Salze, beispielsweise deren Lithium-, Natrium- Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;
   b) Verbindungen vom Typ der (5-Chlor-8-chinolinoxy)malonsäure (S2^{b}), vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.
S3) Verbindungen der Formel (S3) 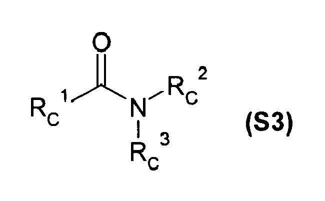 wobei die Symbole und Indizes folgende Bedeutungen haben:
   - R_{c}¹: ist (C₁-C₄)Alkyl, (C₁-C₄)Haloalkyl, (C₂-C₄)Alkenyl, (C₂-C₄)Haloalkenyl, (C₃-C₇)Cycloalkyl, vorzugsweise Dichlormethyl;
   - R_{c}², R_{c}³: sind gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl- (C₁-C₄)alkyl, (C₂-C₄)Alkenylcarbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substitu- iertes oder unsubstituiertes Phenyl, oder R_{c}² und R_{c}³ bilden zusammen einen substituierten oder unsubstituierten heterocyclischen Ring, vorzugsweise ei- nen Oxazolidin-, Thiazolidin-, Piperidin-, Morpholin-, Hexahydropyrimidin- oder Benzoxazinring;
   vorzugsweise:
   Wirkstoffe vom Typ der Dichloracetamide, die häufig als Vorauflaufsafener (bodenwirksame Safener) angewendet werden, wie z. B.
   "Dichlormid" (N,N-Diallyl-2,2-dichloracetamid) (S3-1),
   "R-29148" (3-Dichloracetyl-2,2,5-trimethyl-1,3-oxazolidin) der Firma Stauffer (S3-2),
   "R-28725" (3-Dichloracetyl-2,2,-dimethyl-1,3-oxazolidin) der Firma Stauffer (S3-3),
   "Benoxacor" (4-Dichloracetyl-3,4-dihydro-3-methyl-2H-1,4-benzoxazin) (S3-4), "PPG-1292" (N-Allyl-N-[(1,3-dioxolan-2-yl)-methyl]-dichloracetamid) der Firma PPG Industries (S3-5),
   "DKA-24" (N-Allyl-N-[(allylaminocarbonyl)methyl]-dichloracetamid) der Firma Sagro-Chem (S3-6),
   "AD-67" oder "MON 4660" (3-Dichloracetyl-1-oxa-3-aza-spiro[4,5]decan) der Firma Nitrokemia bzw. Monsanto (S3-7),
   "TI-35" (1-Dichloracetyl-azepan) der Firma TRI-Chemical RT (S3-8),
   "Diclonon" (Dicyclonon) oder "BAS145138" oder "LAB145138" (S3-9) ((RS)-1-Dichloracetyl-3,3,8a-trimethylperhydropyrrolo[1,2-a]pyrimidin-6-on) der Firma BASF,
   "Furilazol" oder "MON 13900" ((RS)-3-Dichloracetyl-5-(2-furyl)-2,2-dimethyloxazolidin) (S3-10); sowie dessen (R)-Isomer (S3-11).
S4) N-Acylsulfonamide der Formel (S4) und ihre Salze, 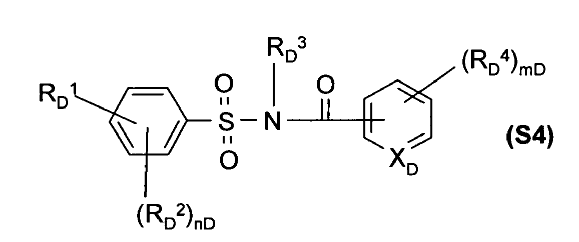 worin die Symbole und Indizes folgende Bedeutungen haben:
   - X_{D}: ist CH oder N;
   - R_{D}¹: ist CO-NR_{D}⁵R_{D}⁶ oder NHCO-R_{D}⁷;
   - R_{D}²: ist Halogen, (C₁-C₄)Haloalkyl, (C₁-C₄)Haloalkoxy, Nitro, (C₁-C₄)Alkyl, (C₁-C₄)Alkoxy, (C₁-C₄)Alkylsulfonyl, (C₁-C₄)Alkoxycarbonyl oder (C₁-C₄)Alkylcarbonyl;
   - R_{D}³: ist Wasserstoff, (C₁-C₄)Alkyl, (C₂-C₄)Alkenyl oder (C₂-C₄)Alkinyl;
   - R_{D}⁴: ist Halogen, Nitro, (C₁-C₄)Alkyl, (C₁-C₄)Haloalkyl, (C₁-C₄)Haloalkoxy, (C₃-C₆)Cycloalkyl, Phenyl, (C₁-C₄)Alkoxy, Cyano, (C₁-C₄)Alkylthio, (C₁-C₄)Alkylsulfinyl, (C₁-C₄)Alkylsulfonyl, (C₁-C₄)Alkoxycarbonyl oder (C₁-C₄)Alkylcarbonyl;
   - R_{D}⁵: ist Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl, Phenyl oder 3- bis 6-gliedriges Heterocyclyl enthaltend V_{D} Heteroatome aus der Gruppe Stickstoff, Sauerstoff und Schwefel, wobei die sieben letztgenannten Reste durch V_{D} Substituenten aus der Gruppe Halogen, (C₁-C₆)Alkoxy, (C₁-C₆)Haloalkoxy, (C₁-C₂)Alkylsulfinyl, (C₁-C₂)Alkylsulfonyl, (C₃-C₆)Cycloalkyl, (C₁-C₄)Alkoxycarbonyl, (C₁-C₄)Alkylcarbonyl und Phenyl und im Falle cyclischer Reste auch (C₁-C₄) Alkyl und (C₁-C₄)Haloalkyl substitu- iert sind;
   - R_{D}⁶: ist Wasserstoff, (C₁-C₆)Alkyl, (C₂-C₆)Alkenyl oder (C₂-C₆)Alkinyl, wobei die drei letztgenannten Reste durch V_{D} Reste aus der Gruppe Halogen, Hydroxy, (C₁-C₄)Alkyl, (C₁-C₄)Alkoxy und (C₁-C₄)Alkylthio substituiert sind, oder
   - R_{D}⁵ und R_{D}⁶: gemeinsam mit dem dem sie tragenden Stickstoffatom einen Pyrrolidi- nyl- oder Piperidinyl-Rest bilden;
   - R_{D}⁷: ist Wasserstoff, (C₁-C₄)Alkylamino, Di-(C₁-C₄)alkylamino, (C₁-C₆)Alkyl, (C₃-C₆)Cycloalkyl, wobei die 2 letztgenannten Reste durch V_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
   - n_{D}: ist 0, 1 oder 2;
   - m_{D}: ist 1 oder 2;
   - V_{D}: ist 0,1,2 oder 3;
   davon bevorzugt sind Verbindungen von Typ der N-Acylsulfonamide, z.B. der nachfolgenden Formel (S4^{a}), die z. B. bekannt sind aus WO-A-97/45016 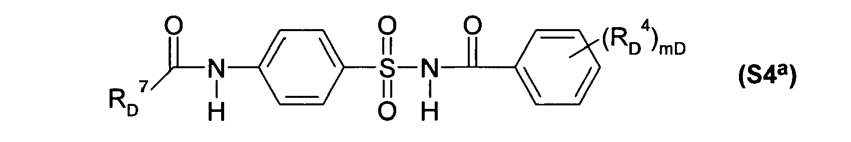 worin
   - R_{D}⁷: (C₁-C₆)Alkyl, (C₃-C₆)Cycloalkyl, wobei die 2 letztgenannten Reste durch V_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
   - R_{D}⁴: Halogen, (C₁-C₄)Alkyl, (C₁-C₄)Alkoxy, CF₃;
   - m_{D}: 1 oder 2;
   - V_{D}: ist 0,1,2 oder 3 bedeutet;
   sowie
   Acylsulfamoylbenzoesäureamide, z.B. der nachfolgenden Formel (S4^{b}), die z.B. bekannt sind aus WO-A-99/16744, 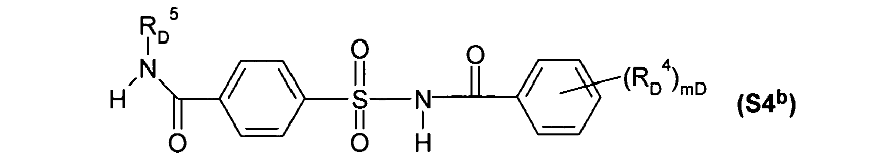 z.B. solche worin
   R_{D}⁵ = Cyclopropyl und (R_{D}⁴) = 2-OMe ist ("Cyprosulfamide", S4-1),
   R_{D}⁵ = Cyclopropyl und (R_{D}⁴) = 5-Cl-2-OMe ist (S4-2),
   R_{D}⁵ = Ethyl und (R_{D}⁴) = 2-OMe ist (S4-3),
   R_{D}⁵ = Isopropyl und (R_{D}⁴) = 5-Cl-2-OMe ist (S4-4) und
   R_{D}⁵= Isopropyl und (R_{D}⁴) = 2-OMe ist (S4-5).
   sowie
   Verbindungen vom Typ der N-Acylsulfamoylphenylharnstoffe der Formel (S4^{c}), die z.B. bekannt sind aus der EP-A-365484, 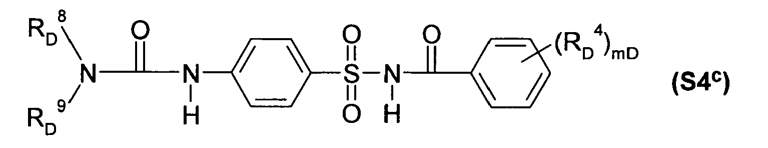 worin
   R_{D}⁸ und R_{D}⁹ unabhängig voneinander Wasserstoff, (C₁-C₈)Alkyl, (C₃-C₈)Cycloalkyl, (C₃-C₆)Alkenyl, (C₃-C₆)Alkinyl,
   - R_{D}⁴: Halogen, (C₁-C₄)Alkyl, (C₁-C₄)Alkoxy, CF₃
   - m_{D}: 1 oder 2 bedeutet;
   beispielsweise
   1-[4-(N-2-Methoxybenzoylsulfamoyl)phenyl]-3-methylharnstoff,
   1-[4-(N-2-Methoxybenzoylsulfamoyl)phenyl]-3,3-dimethylharnstoff,
   1-[4-(N-4,5-Dimethylbenzoylsulfamoyl)phenyl]-3-methylharnstoff.
S5) Wirkstoffe aus der Klasse der Hydroxyaromaten und der aromatischaliphatischen Carbonsäurederivate (S5), z.B.
   3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 2,4-Dichlorzimtsäure, wie sie in der WO-A-2004/084631, WO-A-2005/015994, WO-A-2005/016001 beschrieben sind.
S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydrochinoxalin-2-on-hydrochlorid, 1-(2-Methylsulfonylaminoethyl)-3-(2-thienyl)-1,2-dihydro-chinoxalin-2-on, wie sie in der WO-A-2005/112630 beschrieben sind.
S7) Verbindungen der Formel (S7),wie sie in der WO-A-1998/38856 beschrieben sind 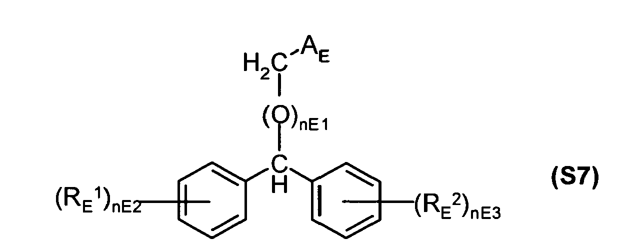 worin die Symbole und Indizes folgende Bedeutungen haben:
   - R_{E}¹, R_{E}²: sind unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
   - A_{E}: ist COOR_{E}³ oder COSR_{E}⁴
   - R_{E}³, R_{E}⁴: sind unabhängig voneinander Wasserstoff, (C₁-C₄)Alkyl, (C₂-C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
   - n_{E}¹: ist 0 oder 1
   - n_{E}², n_{E}³: sind unabhängig voneinander 0, 1 oder 2,
   vorzugsweise:
   Diphenylmethoxyessigsäure,
   Diphenylmethoxyessigsäureethylester,
   Diphenylmethoxyessigsäuremethylester (CAS-Reg.Nr. 41858-19-9) (S7-1).
S8) Verbindungen der Formel (S8), wie sie in der WO-A-98/27049 beschrieben sind 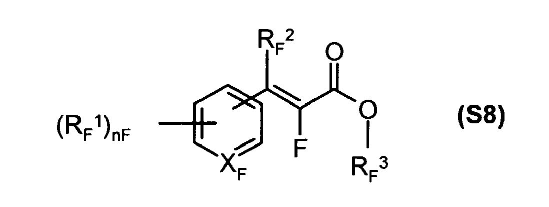 worin
   - X_{F}: CH oder N,
   - n_{F}: für den Fall, dass X_{F}=N ist, eine ganze Zahl von 0 bis 4 und für den Fall, dass X_{F}=CH ist, eine ganze Zahl von 0 bis 5 ,
   - R_{F}¹: Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)-Alkylsulfonyl, (C₁-C₄)Alkoxycarbonyl, ggf. sub- stituiertes. Phenyl, ggf. substituiertes Phenoxy,
   - R_{F}²: Wasserstoff oder (C₁-C₄)Alkyl
   - R_{F}³: Wasserstoff, (C₁-C₈)Alkyl, (C₂-C₄)Alkenyl, (C₂-C₄)Alkinyl, oder Aryl, wobei je- der der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist;bedeuten, oder de- ren Salze,
   vorzugsweise Verbindungen worin
   - X_{F}: CH,
   - n_{F}: eine ganze Zahl von 0 bis 2,
   - R_{F}¹: Halogen, (C₁-C₄)Alkyl, (C₁-C₄)Haloalkyl, (C₁-C₄)Alkoxy, (C₁-C₄)Haloalkoxy,
   - R_{F}²: Wasserstoff oder (C₁-C₄)Alkyl,
   - R_{F}³: Wasserstoff, (C₁-C₈)Alkyl, (C₂-C₄)Alkenyl, (C₂-C₄)Alkinyl, oder Aryl, wobei je- der der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist, bedeuten, oder deren Salze.
S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg.Nr. 219479-18-2), 1,2-Dihydro-4-hydroxy-1-methyl-3-(5-tetrazolylcarbonyl)-2-chinolon (CAS-Reg.Nr. 95855-00-8), wie sie in der WO-A-1999/000020 beschrieben sind.
S10) Verbindungen der Formeln (S10^{a}) oder (S10^{b})
   wie sie in der WO-A-2007/023719 und WO-A-2007/023764 beschrieben sind 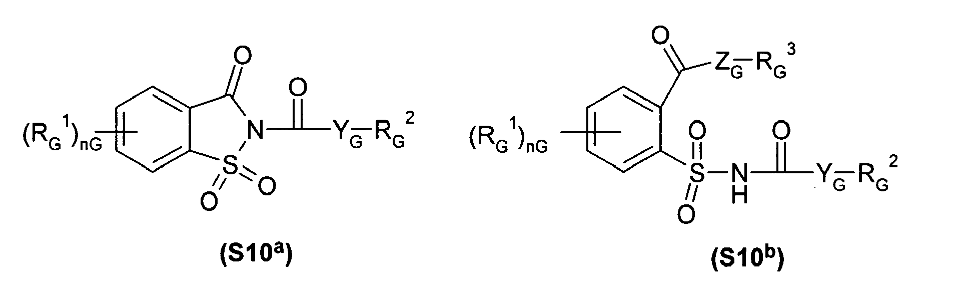 worin
   - R_{G}¹: Halogen, (C₁-C₄)Alkyl, Methoxy, Nitro, Cyano, CF₃, OCF₃
   - Y_{G}, Z_{G}: unabhängig voneinander O oder S,
   - n_{G}: eine ganze Zahl von 0 bis 4,
   - R_{G}²: (C₁-C₁₆)Alkyl, (C₂-C₆)Alkenyl, (C₃-C₆)Cycloalkyl, Aryl; Benzyl, Halogenbenzyl,
   - R_{G}³: Wasserstoff oder (C₁-C₆)Alkyl bedeutet.
S11) Wirkstoffe vom Typ der Oxyimino-Verbindungen (S11), die als Saatbeizmittel bekannt sind, wie z. B.
   "Oxabetrinil" ((Z)-1,3-Dioxolan-2-ylmethoxyimino(phenyl)acetonitril) (S11-1), das als Saatbeiz-Safener für Hirse gegen Schäden von Metolachlor bekannt ist,
   "Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und
   "Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
S12) Wirkstoffe aus der Klasse der Isothiochromanone (S12), wie z.B. Methyl-[(3-oxo-1H-2-benzothiopyran-4(3H)-yliden)methoxy]acetate (CAS-Reg.Nr. 205121-04-6) (S12-1) und verwandte Verbindungen aus WO-A-1998/13361.
S13) Eine oder mehrere Verbindungen aus Gruppe (S13):
   "Naphthalic anhydrid" (1,8-Naphthalindicarbonsäureanhydrid) (S13-1), das als Saatbeiz-Safener für Mais gegen Schäden von Thiocarbamatherbiziden bekannt ist,
   "Fenclorim" (4,6-Dichlor-2-phenylpyrimidin) (S13-2), das als Safener für Pretilachlor in gesätem Reis bekannt ist,
   "Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
   "CL 304415" (CAS-Reg.Nr. 31541-57-8)
   (4-Carboxy-3,4-dihydro-2H-1-benzopyran-4-essigsäure) (S13-4) der Firma American Cyanamid, das als Safener für Mais gegen Schäden von Imidazolinonen bekannt ist,
   "MG 191" (CAS-Reg.Nr. 96420-72-3) (2-Dichlormethyl-2-methyl-1,3-dioxolan)
   (S13-5) der Firma Nitrokemia, das als Safener für Mais bekannt ist,
   "MG-838" (CAS-Reg.Nr. 133993-74-5)
   (2-propenyl 1-oxa-4-azaspiro[4.5]decane-4-carbodithioate) (S13-6) der Firma Nitrokemia,
   "Disulfoton" (O,O-Diethyl S-2-ethylthioethyl phosphordithioat) (S13-7),
   "Dietholate" (O,O-Diethyl-O-phenylphosphorotioat) (S13-8),
   "Mephenate" (4-Chlorphenyl-methylcarbamat) (S13-9).
S14) Wirkstoffe, die neben einer herbiziden Wirkung gegen Schadpflanzen auch Safenerwirkung an Kulturpflanzen wie Reis aufweisen, wie z. B.
   "Dimepiperate" oder "MY-93" (S-1-Methyl-1-phenylethyl-piperidin-1-carbothioat), das als Safener für Reis gegen Schäden des Herbizids Molinate bekannt ist,
   "Daimuron" oder "SK 23" (1-(1-Methyl-1-phenylethyl)-3-p-tolyl-harnstoff), das als Safener für Reis gegen Schäden des Herbizids Imazosulfuron bekannt ist,
   "Cumyluron" = "JC-940" (3-(2-Chlorphenylmethyl)-1-(1-methyl-1-phenylethyl)harnstoff, siehe JP-A-60087254), das als Safener für Reis gegen Schäden einiger Herbizide bekannt ist,
   "Methoxyphenon" oder "NK 049" (3,3'-Dimethyl-4-methoxy-benzophenon), das als Safener für Reis gegen Schäden einiger Herbizide bekannt ist,
   "CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
S15) Wirkstoffe, die vorrangig als Herbizide eingesetzt werden, jedoch auch Safenerwirkung auf Kulturpflanzen aufweisen, z.B.
   (2,4-Dichlorphenoxy)essigsäure (2,4-D),
   (4-Chlorphenoxy)essigsäure,
   (R,S)-2-(4-Chlor-o-tolyloxy)propionsäure (Mecoprop),
   4-(2,4-Dichlorphenoxy)buttersäure (2,4-DB),
   (4-Chlor-o-tolyloxy)essigsäure (MCPA),
   4-(4-Chlor-o-tolyloxy)buttersäure,
   4-(4-Chlorphenoxy)buttersäure,
   3,6-Dichlor-2-methoxybenzoesäure (Dicamba),
   1-(Ethoxycarbonyl)ethyl-3,6-dichlor-2-methoxybenzoat (Lactidichlor-ethyl).

Einige der Safener sind bereits als Herbizide bekannt und entfalten somit neben der Herbizidwirkung bei Schadpflanzen zugleich auch Schutzwirkung bei den Kulturpflanzen.

Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200:1 bis 1:200, vorzugsweise 100:1 bis 1:100, insbesondere 20:1 bis 1:20. Die Safener können analog den Verbindungen der Formel (I) oder deren Mischungen mit weiteren Herbiziden/Pestiziden formuliert werden und als Fertigformulierung oder Tankmischung mit den Herbiziden bereitgestellt und angewendet werden.

Zur Anwendung werden die in handelsüblicher Form vorliegenden Formulierungen gegebenenfalls in üblicher Weise verdünnt z. B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der allgemeinen Formel (I). Sie kann innerhalb weiter Grenzen schwanken, z. B. zwischen 0,001 und 10,0 kg/ha oder mehr Aktivsubstanz, vorzugsweise liegt sie jedoch zwischen 0,005 und 5 kg/ha.

Die vorliegende Erfindung wird an Hand der nachfolgenden Beispiele näher erläutert, welche die Erfindung jedoch keinesfalls beschränken.

### A. Chemische Beispiele

### Herstellung von N2-(5,7-Dimethyl-1,2,3,4-tetrahydro-1-naphthalenyl)-4,6-dimethyl-1,3,5-triazin-2-amine (Tabellenbeispiel Nr. I-17)

Zu einer auf 10 °C gekühlten Lösung aus 21,2 g (0,528 mol) feste Natronlauge in 200 ml Methanol werden unter Rühren portionsweise 50 g (0,528 mol) Acetamidin-Hydrochlorid gegeben. Anschließend werden unter Rühren zu der Suspension 76,4 g (0,528 mol) Trichloracetonitril hinzugetropft. Die Mischung wird für weitere drei Stunden gerührt und ca. 15 Stunden stehen gelassen. Das ausgefallenen Kochsalz wird abgesaugt, die Reaktionsmischung eingeengt und auf Wasser gegeben. Nach Absaugen erhält man 59,5 g Acetimidoyl-2,2,2-trichloro-acetimidoyl-amin (Ausbeute 52 % bei 95 % Reinheit).

Zu einer auf 0 °C gekühlten Lösung aus 40,5 g (0.200 mol) Acetimidoyl-2,2,2-trichloro-acetimidoyl-amin in 100 ml Diethylether werden unter Rühren eine Mischung aus 20,4 g (0.200 mol) Essigsäureanhydrid in 100 ml Diethylether hinzugetropft. Anschließend wird die Mischung 20 Minuten auf Rückfluss erhitzt. Nach Abkühlen wird die Mischung auf Wasser gegeben. Nach Extraktion mit Essigester, Trocknen der organischen Phase und Einengen erhält man 17,4 g 2,4-Dimethyl-6-(trichloromethyl)-1,3,5-triazin als Feststoff mit einem Schmelzpunkt von 72 - 73 °C (Ausbeute 37 % bei 95 % Reinheit).

2,20 g, (9,7 mmol) 2,4-Dimethyl-6-(trichloromethyl)-1,3,5-triazine, 2,06 g (9,7 mmol) 5,7-dimethyl-1,2,3,4-tetrahydronaphthalen-1-aminium chloride und 1,61 g (11,7 mmol) Kaliumcarbonat in 10 ml 1.4-Dioxan werden unter Rühren 4 Stunden auf 100 °C erhitzt, das Rohgemisch wird eingeengt, das verbleibende Rohgemisch auf Kieselgel aufgezogen und säulenchromatographisch mit Heptan/Essigester als Laufmittel gereinigt. Nach Einengen werden 0,6 g N2-(5,7-dimethyl-1,2,3,4-tetrahydro-1-naphthalenyl)-4,6-dimethyl-1,3,5-triazin-2-amine (wachsartig) erhalten (Ausbeute 20 % bei 90 % Reinheit).

### Herstellung von 4,6-Dimethyl-N-[(1R)-1,2,3,4-tetrahydronaphthalen-1-yl]-1,3,5-triazin-2-amine (Tabellenbeispiel Nr. I-78)

0,40 g, (1,8 mmol) 2,4-Dimethyl-6-(trichloromethyl)-1,3,5-triazin und 0,29 g (2.0 mmol) (R)-1,2,3,4-Tetrahydro-1-naphtylamin werden in 1 ml N-Methylpyrrolidon unter Rühren in einer verschlossenen Küvette in einem Mikrowellengerät (Fa. Biotage) 30 Minuten auf 140°C erhitzt, das Rohgemisch wird auf Kieselgel aufgezogen und säulenchromatographisch mit Heptan/Essigester als Laufmittel gereinigt. Nach Einengen werden 0,34 g 4,6-Dimethyl-N-[(1R)-1,2,3,4-tetrahydronaphthalen-1-yl]-1,3,5-triazin-2-amin (fest) erhalten (Ausbeute 72 % bei 95 % Reinheit).

### Herstellung von 4,6-Dimethyl-N-(4-methyl-2,3-dihydro-1H-inden-1-yl)-1,3,5-triazin-2-amine (Tabellenbeispiel Nr. I-168)

0,25 g, (1,1 mmol) 2,4-Dimethyl-6-(trichloromethyl)-1,3,5-triazin, 0,22 g (1.2 mmol) 4-methylindan-1-aminiumchlorid und 0,46 g (2,2 mmol) Dicyclohexylethylamin werden in 1 ml N-Methylpyrrolidon unter Rühren in einer verschlossenen Küvette in einem Mikrowellengerät (Fa. Biotage) 30 Minuten auf 150°C erhitzt, das Rohgemisch wird auf Kieselgel aufgezogen und säulenchromatographisch mit Heptan/Essigester als Laufmittel gereinigt. Nach Einengen werden 0,124 g 4,6-Dimethyl-N-(4-methyl-2,3-dihydro-1H-inden-1-yl)-1,3,5-triazin-2-amine (fest) erhalten (Ausbeute 40 % bei 90 % Reinheit).

### Herstellung von N2-(6-Ethyl-3,4-dihydro-2H-4-chromenyl)-4-methyl-6-(trichlormethyl)-1,3,5-triazin-2-amin (Tabellenbeispiel Nr. I-45)

1,65 g, (5,0 mmol) 2-Methyl-4,6-bis(trichloromethyl)-1,3,5-triazin, 1,07 g (1.2 mmol) 6-Ethylchroman-4-aminium chlorid und 0,79 g (5,5 mmol) Kaliumcarbonat werden in 6 ml Dimethylformamid unter Rühren 240 Minuten auf 120 °C erhitzt. Das abgekühlte Rohgemisch wird auf Wasser gegeben. Das Produkt wird mit Essigester extrahiert, die organische Phase mit Natriumsulfat getrocknet anschließend eingeengt. Das Rohgemisch säulenchromatographisch mit Heptan/Essigester als Laufmittel gereinigt. Nach Einengen werden 2,2 g N2-(6-Ethyl-3,4-dihydro-2H-4-chromenyl)-4-methyl-6-(trichloromethyl)-1,3,5-triazin-2-amine (wachsartig) erhalten (Ausbeute 93 % bei 90 % Reinheit).

### Herstellung von N-[(1R,2S)-2,6-Dimethyl-2,3-dihydro-1H-inden-1-yl]-4-methyl-6-(trichloromethyl)-1,3,5-triazin-2-amin (Tabellenbeispiel Nr. I-70)

0,20 g, (0.6 mmol) 2-Methyl-4,6-bis(trichloromethyl)-1,3,5-triazin und 0,11 g (0.6 mmol) (1 R,2S)-2,6-Dimethyl-2,3-dihydro-1H-inden-1-amin werden in 1 ml N-Methylpyrrolidone unter Rühren in einer verschlossenen Küvette in einem Mikrowellengerät (Fa. Biotage) 20 Minuten auf 120°C erhitzt, das Rohgemisch wird auf Kieselgel aufgezogen und säulenchromatographisch mit Heptan/Essigester als Laufmittel gereinigt. Nach Einengen werden 0,15 g N-[(1R,2S)-2,6-Dimethyl-2,3-dihydro-1 Hinden-1-yl]-4-methyl-6-(trichloromethyl)-1,3,5-triazin-2-amin (wachsartig) erhalten (Ausbeute 61 % bei 90 % Reinheit).

### Herstellung von N-[trans-6-Brom-2-methyl-2,3-dihydro-1H-inden-1-yl]-4-(difluoromethyl)-6-methyl-1,3,5-triazin-2-amin (Tabellenbeispiel Nr. I-235)

Zu einer auf 0 °C gekühlten Lösung aus 7,5 g (0,037 mol) Acetimidoyl-2,2,2-trichloroacetimidoyl-amin in 50 ml Diethylether werden unter Rühren eine Mischung aus 10,0 g (0,200 mol) Difluoressigsäureanhydrid in 50 ml Diethylether hinzugetropft. Anschließend wird die Mischung 20 Minuten auf Rückfluss erhitzt. Nach Abkühlen wird die Mischung auf Wasser gegeben. Nach Extraktion mit Essigester, Trocknen der organischen Phase und Einengen erhält man 8,7 g 2-(difluoromethyl)-4-methyl-6-(trichloromethyl)-1,3,5-triazine (flüssig, Ausbeute 72 % bei 80 % Reinheit).

0,6 g (1,8 mmol) 2-(Difluoromethyl)-4-methyl-6-(trichloromethyl)-1,3,5-triazin (80 % Reinheit), 0,58 g (2.2 mmol) trans 6-Brom-2-methylindan-1-aminium chlorid und 0,58 g (2,8 mmol) Dicyclohexylethylamin werden in 1 ml N-Methylpyrrolidon unter Rühren in einer verschlossenen Küvette in einem Mikrowellengerät (Fa. Biotage) 30 Minuten auf 150°C erhitzt, das Rohgemisch wird auf Kieselgel aufgezogen und säulenchromatographisch mit Heptan/Essigester als Laufmittel gereinigt. Nach Einengen werden 0,257 g N-[trans-6-bromo-2-methyl-2,3-dihydro-1H-inden-1-yl]-4-(difluoromethyl)-6-methyl-1,3,5-triazin-2-amine (wachsartig) erhalten (Ausbeute 34 % bei 90 % Reinheit).

### Herstellung von N-[(1R,2S)-2,6-Dimethyl-2,3-dihydro-1H-inden-1-yl]-4-(methoxymethyl)-6-methyl-1,3,5-triazin-2-amin (Tabellenbeispiel Nr. I-54)

0,20 g, (1,1 mmol) 2-(Methoxymethyl)-4-methyl-6-(methylsulfanyl)-1,3,5-triazine und 0,70 g (4,3 mmol) (1R,2S)-2,6-Dimethyl-2,3-dihydro-1H-inden-1-amin werden in 1 ml N,N-Dimethylacetamid unter Rühren in einer verschlossenen Küvette in einem Mikrowellengerät (Fa. Biotage) 150 Minuten auf 180°C erhitzt, das Rohgemisch wird auf Kieselgel aufgezogen und säulenchromatographisch mit Heptan/Essigester als Laufmittel gereinigt. Nach Einengen werden 0,11 g N-[(1R,2S)-2,6-Dimethyl-2,3-dihydro-1H-inden-1-yl]-4-(methoxymethyl)-6-methyl-1,3,5-triazin-2-amin (wachsartig) erhalten (Ausbeute 31 % bei 95 % Reinheit).

### Chemisch-physikalische Daten:

| Verbindung | Beschreibung |
|---|---|
| I-1 | fest; Schmelzpunkt: 112 - 113°C; 1H-NMR (CDCl₃, 200 MHZ, ö in ppm): 1.90 (m, 3H, 1H von CH₂; CH₂); 2.10 (m, 1H, 1H von CH₂); 2.20 und 2.25 (2*s, 6H, CH₃); 2.40 und 2.60 (2*s, 3H, CH₃); 2.60 (m, 2H, CH₂); 5.30 (m, 1H, CH); 6.20 (dd, 1H, NH); 6.90 (br, 2H, Ar-H) |
| I-5 | fest; Schmelzpunkt: 118 - 119°C; 1H-NMR (CDCl₃, 300 MHZ, ö in ppm): 1.90 (m, 1H, 1H von CH₂); 2.50 und 2.60 (2*s, 3H, CH₃); 2.70 (m, 1H von CH₂); 2.90 (m, 1H von CH₂); 3.00 (m, 1H von CH₂); 5.70 (m, 1H, CH); 5.90 (dd, 1H, NH); 7.10 - 7.40 (m, 4H, Ar-H) |
| I-6 | fest; Schmelzpunkt: 118 - 119°C; 1H-NMR (CDCl₃, 200 MHZ, ö in ppm): 1.90 (m, 1H, 1H von CH₂); 2.30 (s, 3H, CH₃); 2.50 und 2.60 (2*s, 3H, CH₃); 2.70 (m, 1H von CH₂); 2.85 (m, 1H von CH₂); 3.00 (m, 1H von CH₂); 5.70 (m, 1H, CH); 5.90 (dd, 1H, NH); 7.10 - 7.40 (m, 3H, Ar-H) |
| I-7 | fest; Schmelzpunkt: 128 - 130°C; 1H-NMR (CDCl₃, 200 MHZ, δ in ppm): 2.10 (m, 1H, 1H von CH₂); 2.30 (m, 1H, 1H von CH₂); 2.50 und 2.60 (2*s, 3H, CH₃); 4.10 - 4.30 (m, 2H von O-CH₂); 5.30 (m, 1H, CH); 5.90 (br, 1H, NH); 6.80 - 7.00 (m, 3H, Ar-H) |
| I-8 | fest; Schmelzpunkt: 139 - 141°C; 1 H-NMR (CDCl₃, 200 MHZ, δ in ppm): 1.90 (m, 1H, 1H von CH₂); 2.30 und 2.50 (2*s, 6H, CH₃); 2.70 (m, 1H von CH₂); 2.90 (m, 2H von CH₂); 5.60 (dd, 1H, NH); 5.70 (m, 1H, CH); 7.10 - 7.40 (m, 4H, Ar-H) |
| I-16 | wachsartig; 1H-NMR (CDCl₃, 200 MHZ, ö in ppm): 1.30 (m, 3H, CH₃); 1.90 (m, 3H, 1H von CH₂; CH₂); 2.10 (m, 1H, 1H von CH₂); 2.20 und 2.25 (2*s, 6H, CH₃); 2.40 und 2.60 (2*s, 3H, CH₃); 2.60 (m, 2H, CH₂); 2.70 (m, 2H, CH₂); 5.30 (m, 1H, CH); 5.60 (br, 1H, NH); 6.80 (s, 1H, Ar-H); 6.90 (s, 1H, Ar-H); |
| I-17 | wachsartig; 1H-NMR (CDCl₃, 200 MHZ, δ in ppm): 1.90 (m, 3H, 1H von CH₂; CH₂); 2.10 (m, 1H, 1H von CH₂); 2.15 (s, 3H, CH₃); 2.20 (s, 6H, CH₃); 2.40 (s, 3H, CH₃); 2.60 (m, 2H, CH₂); 5.30 (m, 1H, CH); 5.60 (br, 1H, NH); 6.80 (s, 1H, Ar-H); 6.90 (s, 1H, Ar-H); |
| I-18 | fest; Schmelzpunkt: 118 - 120 °C; 1H-NMR (CDCl₃, 200 MHZ, ö in ppm): 2.10 (m, 1H, 1H von CH₂); 2.30 (m, 1H, 1H von CH₂); 2.40 und 2.55 (2*s, 3H, CH₃); 4.10 - 4.30 (m, 2H von O-CH₂); 5.30 (m, 1H, CH); 5.60 (d, 1H, NH); 6.80 - 7.00 (m, 3H, Ar-H) |
| I-19 | fest; 1H-NMR (CDC₃, 200 MHZ, ö in ppm): 1.30 (m, 3H, CH₃); 1.90 (m, 3H, 1H von CH₂; CH₂); 2.10 (m, 1H, 1H von CH₂); 2.25 und 2.45 (2*s, 3H, CH₃); 2.60 (m, 2H, CH₂); 2.70 (m, 2H, CH₂); 5.30 (m, 1H, CH); 5.80 (dd, 1H, NH); 7.00 - 7.30 (m, 4H, Ar-H); |
| I-33 | fest; 1H-NMR (CDCl₃, 200 MHZ, ö in ppm): 1.20 (2*d, 6H, CH₃); 1.90 (m, 1H, 1H von CH₂); 2.40 und 2.50 (2*s, 3H, CH₃); 2.70 (m, 1H von CH₂); 2.80 (m, 1H von ipr-CH); 2.90 (m, 2H von CH₂); 5.60 (dd, 1H, NH); 5.70 (m, 1H, CH); 7.10 - 7.40 (m, 4H, Ar-H) |
| I-34 | wachsartig; 1H-NMR (CDCl₃, 200 MHZ, δ in ppm): 1.30 (2*d, 6H, CH₃); 1.90 (m, 3H, 1H von CH₂; CH₂); 2.10 (m, 1H, 1H von CH₂); 2.20 (s, 3H, CH₃); 2.25 (s, 3H, CH₃); 2.40 und 2.50 (2*s, 3H, CH₃); 2.60 (m, 2H, CH₂); 2.80 (m, 1H von ipr-CH); 5.30 (m, 1H, CH); 5.50 (br, 1H, NH); 6.90 (s, 1H, Ar-H); 7.00 (s, 1H, Ar-H); |
| I-35 | wachsartig; 1H-NMR (CDCl₃, 200 MHZ, ö in ppm): 1.25 (2*d, 6H, CH₃); 1.90 (m, 3H, 1H von CH₂; CH₂); 2.10 (m, 1H, 1H von CH₂); 2.35 und 2.50 (2*s, 3H, CH₃); 2.75 (m, 2H, CH₂); 2.80 (m, 1H von ipr-CH); 5.40 (m, 1H, CH); 5.50 (br, 1H, NH); 7.10 - 7.40 (m, 4H, Ar-H); |
| I-36 | wachsartig; 1H-NMR (CDCl₃, 200 MHZ, δ in ppm): 1.25 (2*d, 6H, CH₃); 2.20 (m, 2H, CH₂); 2.40 und 2.50 (2*s, 3H, CH₃); 2.80 (m, 1H von ipr-CH); 4.20 (m, 2H, O-CH₂); 5.30 (m, 1H, CH); 5.50 (br, 1 H, NH); 6.80 - 6.95 (m, 2H, Ar-H); 7.10 - 7.30 (m, 2H, Ar-H); |
| I-37 | wachsartig; 1H-NMR (CDCl₃, 200 MHZ, ö in ppm): 1.90 (m, 1H, 1H von CH₂); 2.50 und 2.60 (2*s, 3H, CH₃); 2.70 (m, 1H von CH₂); 2.90 (m, 2H von CH₂); 5.70 (m, 1H, CH); 5.80 (br, 1H, NH); 7.10 - 7.40 (m, 4H, Ar-H) |
| I-38 | wachsartig; 1H-NMR (CDCl₃, 200 MHZ, ö in ppm): 1.20 (m, 4H, 2*cp-CH₂); 1.90 (m, 1H, 1H von CH₂); 2.00 (m, 1H, cp-CH); 2.70 (m, 1H von CH₂); 2.90 (m, 2H von CH₂); 5.70 (m, 1H, CH); 5.80 (br, 1H, NH); 7.10 - 7.40 (m, 4H, Ar-H) |
| I-39 | wachsartig; 1H-NMR (CDCl₃, 200 MHZ, ö in ppm): 1.25 (2*d, 6H, CH₃); 1.90 (m, 1H, 1H von CH₂); 2.70 (m, 1H von CH₂); 2.85 (m, 1H von ipr-CH); 2.95 (m, 2H von CH₂); 5.70 (m, 1H, CH); 5.90 (br, 1H, NH); 7.10 - 7.40 (m, 4H, Ar-H) |
| I-40 | fest; 1H-NMR (CDCl₃, 200 MHZ, ö in ppm): 1.25 (2*d, 6H, CH₃); 2.20 (m, 2H, CH₂); 3.00 (m, 1H von ipr-CH); 4.20 (m, 2H, O-CH₂); 5.30 (m, 1H, CH); 5.90 (br, 1H, NH); 6.80 - 6.95 (m, 2H, Ar-H); 7.10 - 7.30 (m, 2H, Ar-H); |
| I-41 | fest; 1H-NMR (CDCl₃, 200 MHZ, ö in ppm): 1.25 (2*d, 6H, CH₃); 1.90 (m, 4H, 2*2H von CH₂); 2.20 (s, 3H, CH₃); 2.25 (s, 3H, CH₃); 2.75 (m, 2H von CH₂); 2.95 (m, 1H von ipr-CH); 5.35 (m, 1H, CH); 5.90 (br, 1H, NH); 6.90 (d, 1H, Ar-H); 7.05 (d, 1H, Ar-H); |
| I-42 | wachsartig; 1H-NMR (CDCl₃, 200 MHZ, ö in ppm): 1.25 (2*d, 6H, CH₃); 1.90 (m, 4H, 2*2H von CH₂); 2.20 (s, 3H, CH₃); 2.25 (s, 3H, CH₃); 2.40 und 2.50 (2*s, 3H, CH₃); 2.75 (m, 2H von CH₂); 2.95 (m, 1H von ipr-CH); 5.35 (m, 1H, CH); 5.50 (br, 1H, NH); 6.90 (br, 1H, Ar-H); 7.05 (br, 1H, Ar-H); |
| I-43 | wachsartig; 1H-NMR (CDCl₃, 200 MHZ, δ in ppm): 1.25 (2*d, 6H, CH₃); 1.60 - 2.00 (m, 6H, 3*2H von CH₂); 2.20 (s, 3H, CH₃); 2.25 (s, 3H, CH₃); 2.40 (s, 3H, CH₃); 2.75 (m, 2H von CH₂); 2.95 (m, 1H von ipr-CH); 5.35 (m, 1H, CH); 5.90 (br, 1H, NH); 7.00 - 7.30 (m, 4H, Ar-H) |
| I-44 | wachsartig; 1H-NMR (CDCl₃, 200 MHZ, ö in ppm): 1.70 (2*d, 3H, CH₃); 1.90 (m, 1H, 1H von CH₂); 2.70 (m, 1H von CH₂); 2.95 (m, 2H von CH₂); 5.60 (m, 1H, CHF); 5.70 (m, 1H, CH); 6.10 (br, 1H, NH); 7.10 - 7.40 (m, 4H, ArH) |
| I-45 | wachsartig; 1H-NMR (CDCl₃, 200 MHZ, δ in ppm): 1.20 (2*t, 3H, CH₃); 2.15 (m, 1H, CH₂); 2.25 (m, 1H, CH₂); 2.55 (m, 2H, CH₂); 4.20 (m, 2H, O-CH₂); 5.30 (m, 1H, CH); 5.90 (br, 1H, NH); 6.80 (d, 1H, Ar-H); 7.00 - 7.10 (m, 2H, Ar-H); |
| I-46 | fest; 1H-NMR (CDCl₃, 200 MHZ, ö in ppm): 1.40 und 1.50 (2*s, 6H, 2*CH₃); 1.80 (m, 2H, CH₂); 2.30 (s, 3H, CH₃); 2.50 und 2.60 (2*s, 3H, CH₃); 5.30 (m, 1H, CH); 5.80 (br, 1H, NH); 6.75 - 6.80 (m, 2H, Ar-H); 7.05 (m, 1H, Ar-H); |
| I-47 | wachsartig; 1H-NMR (CDCl₃, 200 MHZ, ö in ppm): 2.60 (2*s, 6H, CH₃); 2.20 - 3.00 (m, 5H, 2*CH₂, CH); 2.50 und 2.60 (2*s, 3H, CH₃); 5.50 (m, 1H, CH); 5.70 (br, 1H, NH); 6.80 - 7.30 (m, 7H, Ar-H) |
| I-48 | fest |
| I-49 | wachsartig |
| I-50 | wachsartig; logp (HCOOH): 2.16; 1H-NMR (CDCl₃, 400 MHZ, δ in ppm): 1.25 (2*d, 9H, 3*CH₃); 2.25 (m, 1H, 1H von CH₂); 2.30 (s, 3H, CH₃); 2.40 und 2.45 (2*s, 3H, CH₃); 2.55 (m, 1H, 1H von CH₂); 2.85 (m, 1H, ipr-CH); 3.05 (dd, 1H, CH); 5.35 (br, 2H, CH; NH); 7.00 - 7.10 (m, 3H, Ar-H); |
| I-51 | fest, Schmelzpunkt 116 - 117°C; logp (HCOOH): 3.56; 1H-NMR (CDCl₃, 400 MHZ, δ in ppm): 1.25 (d, 3H, CH₃); 1.30 (s, 9H, CH₃); 2.25 (m, 1H, 1H von CH₂); 2.30 (s, 3H, CH₃); 2.40 und 2.45 (2*s, 3H, CH₃); 2.55 (m, 1H, 1H von CH₂); 3.05 (dd, 1H, CH); 5.35 (br, 2H, CH; NH); 7.00 - 7.10 (m, 3H, Ar-H); |
| I-52 | wachsartig; logp (HCOOH): 1.85; 1H-NMR (CDCl₃, 400 MHZ, ö in ppm): 1.25 (m, 6H, 2*CH₃); 2.25 (m, 1H, 1H von CH₂); 2.30 (s, 3H, CH₃); 2.40 und 2.45 (2*s, 3H, CH₃); 2.55 (m, 1H, 1H von CH₂); 2.65 und 2.70 (2*q, 2H, CH₂); 3.05 (dd, 1H, CH); 5.35 (br, 2H, CH; NH); 7.00 - 7.10 (m, 3H, Ar-H); |
| I-53 | wachsartig; logp (HCOOH): 1.48; 1H-NMR (CDCl₃, 400 MHZ, δ in ppm): 1.30 (m, 3H, CH₃); 1.90 (m, 3H, 1H von CH₂; CH₂); 2.10 (m, 1H, 1H von CH₂); 2.40 und 2.50 (2*s, 3H, CH₃); 2.60 und 2.70 (2*q, 2H, CH₂); 2.85 (m, 2H, CH₂); 5.40 (m, 1H, CH); 5.50 (br, 1H. NH); 6.30 und 6.35 (2*t, 1H, CHF₂); 7.10 - 7.35 (m, 4H, Ar-H) |
| I-54 | fest; logp (HCOOH): 2.16; 1H-NMR (CDCl₃, 400 MHZ, ö in ppm): 1.25 (d, 3H, CH₃); 2.25 (m, 1H, 1H von CH₂); 2.30 (s, 3H, CH₃); 2.45 (s, 3H, CH₃); 2.55 (m, 1H, 1H von CH₂); 3.05 (dd, 1H, CH); 3.50 (s, 3H, OCH₃); 4.40 (s, 2H, CH₂O); 5.35 (m, 1H, CH); 5.50 (br, 1H, NH₂); 6.95 - 7.10 (m, 3H, Ar-H); |
| I-63 | fest; logp (HCOOH): 1.59; 1H-NMR (CDCl₃, 400 MHZ, δ in ppm): 1.25 (d, 3H, CH₃); 2.25 (m, 1H, 1H von CH₂); 2.30 (s, 3H, CH₃); 2.37 und 2.42 (2*s, 3H, CH₃); 2.55 (m, 1H, 1H von CH₂); 3.05 (dd, 1H, CH); 5.35 (m, 1H, CH); 5.40 (br, 1H, NH₂); 6.95 - 7.10 (m, 3H, Ar-H); |
| I-64 | wachsartig; logp (HCOOH): 3.44; 1H-NMR (CDCl₃, 400 MHZ, ö in ppm): 1.25 (d, 3H, CH₃); 2.30 (s, 3H, CH₃); 2.35 (m, 1H, 1H von CH₂); 2.55 (m, 1H, 1H von CH₂); 2.50 und 2.55 (2*s, 3H, CH₃); 3.10 (dd, 1H, CH); 5.35 (m, 1H, CH); 5.75 (br, 1H, NH₂); 6.30 und 6.32 (2*t, 1H, CHF₂); 6.95 - 7.10 (m, 3H, Ar-H); |
| I-65 | fest; logp (HCOOH): 2.96; 1H-NMR (CDCl₃, 400 MHZ, ö in ppm): 1.90 (m, 3H, 1H von CH₂; CH₂); 2.10 (m, 1H, 1H von CH₂); 2.45 und 2.55 (2*s, 3H, CH₃); 2.85 (m, 2H, CH₂); 5.40 (m, 1H, CH); 5.80 (br, 1H, NH); 6.30 und 6.35 (2*t, 1H, CHF₂); 7.10 - 7.30 (m, 4H, Ar-H) |
| I-66 | wachsartig; logp (HCOOH): 4.36; 1H-NMR (CDCl₃, 400 MHZ, δ in ppm): 1.25 (d, 3H, CH₃); 2.30 (s, 3H, CH₃); 2.35 (m, 1H, 1H von CH₂); 2.55 (m, 1H, 1H von CH₂); 2.50 und 2.55 (2*s, 3H, CH₃); 3.10 (dd, 1H, CH); 5.35 (m, 1H, CH); 5.80 (br, 1H, NH₂); 7.00 - 7.15 (m, 3H, Ar-H); |
| I-67 | wachsartig; logp (HCOOH): 3.90; 1H-NMR (CDCl₃, 400 MHZ, ö in ppm): 1.90 (m, 3H, 1H von CH₂; CH₂); 2.10 (m, 1H, 1H von CH₂); 2.50 und 2.60 (2*s, 3H, CH₃); 2.80 (m, 2H, CH₂); 5.40 (m, 1H, CH); 5.90 (br, 1H, NH); 7.10 - 7.30 (m, 4H, Ar-H) |
| I-68 | wachsartig; logp (HCOOH): 3.65; 1H-NMR (CDCl₃, 400 MHZ, ö in ppm): 1.85 (m, 3H, 1H von CH₂; CH₂); 2.10 (m, 1H, 1H von CH₂); 2.45 und 2.55 (2*s, 3H, CH₃); 2.80 (m, 2H, CH₂); 5.40 (m, 1H, CH); 5.80 (br, 1H, NH); 6.30 und 6.40 (2*s, 1H, CHCl₂); 7.05 - 7.30 (m, 4H, Ar-H) |
| I-69 | wachsartig; logp (HCOOH): 4.13; 1H-NMR (CDCl₃, 400 MHZ, ö in ppm): 1.30 (d, 3H, CH₃); 2,35 (s, 3H, CH₃); 2.35 (m, 1H, 1H von CH₂); 2.55 (m, 1H, 1H von CH₂); 2.55 und 2.60 (2*s, 3H, CH₃); 3.10 (dd, 1H, CH); 5.35 (m, 1H, CH); 5.80 (br, 1H, NH₂); 6.35 und 6.40 (2*s, 1H, CHCl₂); 7.05 - 7.15 (m, 3H, Ar-H); |
| I-70 | wachsartig; logp (HCOOH): 4.85; 1H-NMR (CDCl₃, 400 MHZ, δ in ppm): 1.30 (d, 3H, CH₃); 2,35 (s, 3H, CH₃); 2.35 (m, 1H, 1H von CH₂); 2.55 (m, 1H, 1H von CH₂); 2.55 und 2.60 (2*s, 3H, CH₃); 3.10 (dd, 1H, CH); 5.35 (m, 1H, CH); 5.80 (br, 1H, NH₂); 7.05 - 7.15 (m, 3H, Ar-H); |
| I-71 | fest, Schmelzpunkt 119 - 120 °C; logp (HCOOH): 4.40; 1H-NMR (CDCl₃, 400 MHZ, ö in ppm): 1.85 (m, 3H, 1H von CH₂; CH₂); 2.10 (m, 1H, 1H von CH₂); 2.50 und 2.60 (2*s, 3H, CH₃); 2.80 (m, 2H, CH₂); 5.40 (m, 1H, CH); 5.90 (br, 1H, NH); 7.05 - 7.30 (m, 4H, Ar-H) |
| I-74 | wachsartig; |
| I-76 | wachsartig; logp (HCOOH): 1.76; 1H-NMR (CDCl₃, 400 MHZ, δ in ppm): 1.35 (dd, 6H, 2*3H, 2*CH₃); 1.85 (m, 3H, 1H von CH₂; CH₂); 2.05 (m, 1H, 1H von CH₂); 2.35 und 2.45 (2*s, 3H, CH₃); 2.80 (m, 3H, ipr-CH, CH₂); 5.35 (m, 1H, CH); 5.45 (br, 1H, NH); 7.05 - 7.30 (m, 4H, Ar-H) |
| I-78 | Wachsartig; logp (HCOOH): 1.35; 1H-NMR (CDCl₃, 400 MHZ, ö in ppm): 1.85 (m, 3H, 1H von CH₂; CH₂); 2.05 (m, 1H, 1H von CH₂); 2.35 und 2.45 (2*s, 2*3H, 2*CH₃); 2.80 (m, 2H, CH₂); 5.35 (m, 1H, CH); 5.45 (br, 1H, NH); 7.05 - 7.30 (m, 4H, Ar-H) |
| I-81 | fest; logp (HCOOH): 3.80; 1H-NMR (CDCl₃, 400 MHZ, ö in ppm): 1.30 (d, 3H, CH₃); 2.35 (m, 1H, 1H von CH₂); 2.55 (dd, 1H, 1H von CH₂); 2.55 und 2.60 (2*s, 3H, CH₃); 3.10 (dd, 1H, CH); 5.40 (m, 1H, CH); 5.80 (br, 1H. NH₂); 7.05 (d, 1H, Ar-H); 7.35 (m, 2H, Ar-H); |
| I-82 | wachsartig; logp (HCOOH): 4.32; 1H-NMR (CDCl₃, 400 MHZ, ö in ppm): 1.35 und 1.45 (2*s, 2*3H, 2*CH₃); 1.80 (m, 1H, 1H von CH₂); 2.30 (m, 1H, 1H von CH₂); 2.55 und 2.60 (2*s, 3H, CH₃); 5.45 (m, 1H, CH); 5.75 (br, 1H, NH); 6.85 (d, 1H, AR-H); 7.00 (dd, 1H, AR-H) |
| I-83 | wachsartig; logp (HCOOH): 3.06; 1H-NMR (CDCl₃, 400 MHZ, δ in ppm): 2.20 (m, 1H, 1H von CH₂); 2.30 (m, 1H, 1H von CH₂); 2.50 und 2.60 (2*s, 3H, CH₃); 4.30 (m, 1H, 1H von CH₂); 4.40 (m, 1H, 1H von CH₂); 5.40 (m, 1H, CH); 5.90 (br, 1H, NH); 6.90 (m, 1H, AR-H); 7.00 (m, 2H, AR-H) |
| I-84 | fest; logp (HCOOH): 3.11; 1H-NMR (CDCl₃, 400 MHZ, ö in ppm): 2.15 (m, 1H, 1H von CH₂); 2.30 (m, 1H, 1H von CH₂); 2.50 und 2.60 (2*s, 3H, CH₃); 4.20 (m, 1H, 1H von CH₂); 4.30 (m, 1H, 1H von CH₂); 5.35 (m, 1H, CH); 5.90 (br, 1H, NH); 6.90 (m, 1H, AR-H); 7.20 (m, 2H, AR-H) |
| I-87 | wachsartig; logp (HCOOH): 4.21; 1H-NMR (CDCl₃, 400 MHZ, ö in ppm): 1.25 (d, 2H, CH₃); 2.25 (m, 1H, 1H von CH₂); 2.30 (s, 3H, CH₃); 2.50 und 2.55 (2*s, 3H, CH₃); 2.55 (m, 1H, 1H von CH₂); 3.05 (dd, 1H, CH); 5.40 (m, 1H, CH); 5.80 (br, 1H, NH); 7.00 - 7.15 (m, 3H, Ar-H); |
| I-91 | wachsartig; logp (HCOOH): 2.69; 1H-NMR (CDCl₃, 400 MHZ, ö in ppm): 1.90 (m, 1H, 1H von CH₂); 2.50 und 2.60 (2*s, 3H, CH₃); 2.55 (m, 1H, 1H von CH₂); 2.90 (m, 1H, 1H von CH₂); 3.05 (m, 1H, 1H von CH₂); 5.70 (m, 1H, CH); 5.80 (br, 1H, NH); 6.30 (2*t, 1H, CF₂H); 7.15 - 7.30 (m, 4H, Ar-H); |
| I-95 | wachsartig; logp (HCOOH): 4.32; 1H-NMR (CDCl₃, 400 MHZ, δ in ppm): 1.35 und 1.45 (2*s, 2*3H, 2*CH₃); 1.80 (m, 1H, 1H von CH₂); 2.30 (m, 1H, 1H von CH₂); 2.55 und 2.60 (2*s, 3H, CH₃); 5.45(m, 1H, CH); 5.65 (br, 1H, NH); 6,30 ( 2*t, 1H, CHF₂); 6.85 (d, 1H, AR-H); 7.00 (dd, 1H, Ar-H); |
| I-100 | wachsartig; logp (HCOOH): 3.79; 1H-NMR (CDCl₃, 400 MHZ, ö in ppm): 1.40 (d, 2*3H, 2*CH₃); 1.85 (m, 1H, 1H von CH₂); 2.45 und 2.55 (2*s, 3H, CH₃); 2.65 (m, 1H, 1H von CH₂); 2.80 - 3.05 (m, 3H, 1H, CH(CH₃)₂, 2H von CH₂); 5.65 (m, 1H, CH); 5.75 (br, 1H, NH); 6.30 (2*t, 1H, CF₂H); 7.10 - 7.25 (m, 3H, Ar-H); |
| I-122 | wachsartig; logp (HCOOH): 3.34; 1H-NMR (CDCl₃, 400 MHZ, ö in ppm): 1.35 (t, 3H, CH₃); 1.90 (m, 3H, 3H von CH₂); 2.10 (m, 1H, 1H von CH₂); 2.45 und 2.55 (2*s, 3H, CH₃); 2.75 (m, 2H, 2H von CH₂); 3.95 (m, 2H, 2H von CH₂); 5.35 (m, 1H, CH); 5.80 (br, 1H, NH); 6.30 (2*t, 1H, CF₂H); 6.80 (m, 2H, AR-H); 7.00 (m, 1H, Ar-H); |
| I-125 | wachsartig; logp (HCOOH): 3.41; 1H-NMR (CDCl₃, 400 MHZ, δ in ppm): 1.40 (d, 2*3H, 2*CH₃); 2.10 (m, 1H, 1H von CH₂); 2.30 (m, 1H, 1H von CH₂); 2.50 und 2.55 (2*s, 3H, CH₃); 2.80 (m, 1H, 1H von CH(CH₃)₂); 4.15 (m, 1H, 1H von CH₂O); 4.25 (m, 1H, 1H von CH₂O; 5.30 (m, 1H, CH); 5.85 (br, 1H, NH); 6.30 (2*t, 1H, CF₂H); 6.80 (d, 1H, Ar-H); 7.05 - 7.15 (m, 2H, AR-H); |
| I-130 | wachsartig; logp (HCOOH): 3.30; 1H-NMR (CDCl_{3,} 400 MHZ, ö in ppm): 1.35 (s, 3H, CH₃); 1.40 (s, 3H, CH₃); 1.75 (m, 1H, 1H von CH₂); 2.25 (s, 3H, CH₃); 2.30 (m, 1H, 1H von CH₂); 2.50 und 2.55 (2*s, 3H, CH₃); 5.45 (m, 1H, CH); 5.65 (br, 1H, NH); 6.30 (2*t, 1H, CF₂H); 6.80 (d, 1H, Ar-H); 6.65 (s, 1H, AR-H); 6.70 (d, 1H, Ar-H); 7.10 (d, 1H, Ar-H); |
| I-134 | wachsartig; logp (HCOOH): 3.10; 1H-NMR (CDCl₃, 400 MHZ, δ in ppm): 1.15 (t, 3H, CH₃); 2.10 (m, 1H, 1H von CH₂); 2.30 (m, 1H, 1H von CH₂); 2.50 und 2.55 (2*s, 3H, CH₃); 2.55 (q, 2H, CH₂); 4.15 (m, 1H, 1H von CH₂O); 4.25 (m, 1H, 1H von CH₂O); 5.30 (m, 1H, CH); 5.85 (br, 1H, NH); 6.30 (2*t, 1H, CF₂H); 6.80 (d, 1H, Ar-H); 7.05 - 7.15 (m, 2H, Ar-H); |
| I-135 | wachsartig; logp (HCOOH): 3.71; 1H-NMR (CDCl₃, 400 MHZ, δ in ppm): 1.20 (t, 3H, CH₃); 2.15 (m, 1H, 1H von CH₂); 2.25 (m, 1H, 1H von CH₂); 2.50 - 2.60 (2*s, 3H, CH₃); 2.55 (q, 2H von CH₂-CH₃); 4.15, 4.25 (2*m, 2*1H, 2*1H von CH₂-O); 5.30 (q, 1H, CH); 5.55 (br, 1H, NH₂); 6.80 (d, 1H, Ar-H); 7.00 - 7.10 (m, 2H, Ar-H); |
| I-137 | wachsartig; logp (HCOOH): 3.58; 1H-NMR (CDCl₃, 400 MHZ, δ in ppm): 1.30 (d, 3H, CH₃); 2.35 (m, 1H, 1H von CH₂); 2.50 - 2.55 (2*s, 3H, CH₃); 2.55 (dd, 1H, 1H von CH₂); 3.10 (dd, 1H, CH); 5.40 (m, 1H, CH); 5.80 (br, 1H, NH₂); 6.30 (2*t, 1H, CF₂H); 7.05 - 7.25 (m, 3H, Ar-H); |
| I-141 | wachsartig; 1H-NMR (CDCl₃, 400 MHZ, δ in ppm): 1.00 (m, 3H, CH₃); 1.50 (m, 1H, 1H von CH₂); 1.80 (m, 1H, 1H von CH₂); 2.20 (m, 1H, 1H von CH₂); 2.50 - 2.55 (2*s, 3H, CH₃); 2.60 (dd, 1H, 1H von CH₂); 3.10 (dd, 1H, CH); 5.45 (q, 1H, CH); 5.70 (br, 1H, NH₂); 6.30 (2*t, 1H, CF₂H); 7.10 - 7.25 (m, 4H, Ar-H); |
| I-146 | wachsartig; 1H-NMR (CDCl₃, 400 MHZ, δ in ppm): 1.80 (m, 1H, 1H von CH₂); 2.20 (s, 3H, CH₃); 2.50 - 2.55 (2*s, 3H, CH₃); 2.70 (m, 1H, 1H von CH₂); 2.80 (m, 1H, 1H von CH₂); 2.95 (m, 1H, 1H von CH₂); 5.65 (q, 1H, CH); 5.70 (br, 1H, NH₂); 6.30 (2*t, 1H, CF₂H); 7.10 - 7.25 (m, 3H, Ar-H); |
| I-147 | wachsartig; logp (HCOOH): 3.98; 1H-NMR (CDCl₃, 400 MHZ, δ in ppm): 1.90 (m, 1H, 1H von CH₂); 2.30 (s, 3H, CH₃); 2.50 - 2.55 (2*s, 3H, CH₃); 2.65 (m, 1H, 1H von CH₂); 2.80 (m, 1H, 1H von CH₂); 2.95 (m, 1H, 1H von CH₂); 5.65 (q, 1H, CH); 5.85 (br, 1H, NH₂); 7.10 - 7.25 (m, 3H, Ar-H); |
| I-148 | wachsartig; 1H-NMR (CDCl₃, 400 MHZ, δ in ppm): 1.30 (d, 3H, CH₃); 2.20 (s, 3H, CH₃); 2.25 (m, 1H, 1H von CH₂); 2.50 - 2.55 (2*s, 3H, CH₃); 2.55 (dd, 1H, 1H von CH₂); 3.10 (dd, 1H, CH); 5.40 (m, 1H, CH); 5.80 (br, 1H, NH₂); 6.30 (2*t, 1H, CF₂H); 7.05 - 7.25 (m, 3H, Ar-H); |
| I-151 | wachsartig; 1H-NMR (CDCl₃, 400 MHZ, δ in ppm): 1.90 (m, 1H, 1H von CH₂); 2.50 - 2.55 (2*s, 3H, CH₃); 2.70 (m, 1H, 1H von CH₂); 2.80 (m, 1H, 1H von CH₂); 2.95 (m, 1H, 1H von CH₂); 5.65 (q, 1H. CH); 5.70 (br, 1H, NH₂); 6.30 (2*t, 1H, CF₂H); 7.15 - 7.30 (m, 3H, Ar-H); |
| I-159 | wachsartig; logp (HCOOH): 2.90; 1H-NMR (CDCl₃, 400 MHZ, δ in ppm): 1.85 (m, 3H, 3H von CH₂); 2.05 (m, 1H, 1H von CH₂); 2.50 - 2.55 (2*s, 3H, CH₃); 2.80 (m, 2H, 2H von CH₂); 3.80 (s, 3H, OCH₃); 5.35 (q, 1H, CH); 5.70 (br, 1H, NH₂); 6.30 (2*t, 1H, CF₂H); 6.65 (s, 1H, Ar-H); 6.70 (d, 1H, Ar-H); 7.20 (d, 1H, Ar-H); |
| I-168 | wachsartig; logp (HCOOH): 1.36; 1H-NMR (CDCl₃, 400 MHZ, δ in ppm): 1.85 (m, 1H, 1H von CH₂); 2.30 (m, 1H, 1H von CH₂); 2.30, 2.35, 2.45 (3*s, 3*3H, 3*CH₃); 2.55 (m, 1H, 1H von CH₂); 2.75 (m, 1H, 1H von CH₂); 2.95 (m, 1H, 1H von CH₂); 5.50 (br, 1H, NH₂); 5.70 (q, 1H, CH); 7.00 - 7.15 (m, 3H, Ar-H); |
| I-235 | wachsartig; logp (HCOOH): 3.55; 1H-NMR (CDCl₃, 400 MHZ, ö in ppm): 1.30 (d, 3H, CH₃); 2.25 (m, 1H, 1H von CH₂); 2.50 - 2.55 (2*s, 3H, CH₃); 2.55 (m, 1H, 1H von CH₂); 3.05 (dd, 1H, CH); 5.40 (q, 1H, CH); 5.70 (br, 1H, NH₂); 6.30 (2*t, 1H, CF₂H); 7.10 (d, 1H, Ar-H); 7.25 - 7.35 (m, 2H, Ar-H); |
| I-236 | fest; logp (HCOOH): 3.71; 1H-NMR (CDCl₃, 400 MHZ, δ in ppm): 1.85 (m, 3H, 2H und 1H von CH₂); 2.15 (m, 1H, 1H von CH₂); 2.50 - 2.55 (2*s, 3H, CH₃); 2.80 (m, 2H, 2H von CH₂); 5.40 (q, 1H, CH); 5.85 (br, 1H, NH₂); 7.15 - 7.30 (m, 4H, Ar-H); |
| I-237 | wachsartig; logp (HCOOH): 2.93; 1H-NMR (CDCl₃, 400 MHZ, ö in ppm): 2.10 (m, 1H, 1H von CH₂); 2.30 (m, 1H, 1H von CH₂); 2.50 - 2.55 (2*s, 3H, CH₃); 3.70 (s, 3H, O-CH₃); 4.10, 4.25 (2*m, 2*1 H, 2*1H von CH₂-O); 5.30 (q, 1H, CH); 5.55 (br, 1H, NH₂); 6.75 (d, 1H, Ar-H); 7.00 - 7.10 (m, 2H, Ar-H); |
| I-238 | wachsartig; logp (HCOOH): 1.51; 1H-NMR (CDCl₃, 400 MHZ, ö in ppm): 1.25 (t, 3H, CH₃); 2.10 (m, 1H, 1H von CH₂); 2.30 (m, 1H, 1H von CH₂); 2.35, 2.45 (2*s, 2*3H, 2*CH₃); 2.65 (q, 2H von CH₂-CH₃); 4.10, 4.25 (2*m, 2*1H, 2*1H von CH₂-O); 5.30 (q, 1H, CH); 5.55 (br, 1H, NH₂); 6.75 (d, 1H, Ar-H); 7.00 - 7.10 (m, 2H, Ar-H); |

### B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netzund Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I) und/oder deren Salze,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I) und/oder deren Salze,
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### Versuchsbeschreibung

1. Herbizide Wirkung bzw. Kulturpflanzenverträglichkeit im Vorauflauf Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 I/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen). Dabei zeigen beispielsweise die Verbindungen Nr. I-16, I-17, I-48, I-50, I-51, I-52, I-54 und I-63 bei einer Aufwandmenge von 320 g/ha jeweils eine mindestens 80%-ige Wirkung gegen Lolium multiflorum, Setaria viridis und Stellaria media. Die Verbindungen Nr. I-64, I-65, I-66, I-68, I-69 und I-70 zeigen bei einer Aufwandmenge von 320 g/ha jeweils eine mindestens 80%-ige Wirkung gegen Amaranthus retroflexus, Pharbitis purpureumund Veronica persica.
2. Herbizide Wirkung bzw. Kulturpflanzenverträglichkeit im Nachauflauf Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 I/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen). Dabei zeigen beispielsweise die Verbindungen Nr. I-50, I-52, I-54 und I-64 bei einer Aufwandmenge von 320 g/ha jeweils eine mindestens 80%-ige Wirkung gegen Alopecurus myosuroides, Setaria viridis und Stellaria media. Die Verbindungen Nr. I-51, I-63, I-66, I-69 und I-70 zeigen bei einer Aufwandmenge von 320 g/ha jeweils eine mindestens 80%-ige Wirkung gegen Alopecurus myosuroides, Veronica persica und Viola tricolor.

## Patentansprüche

1. Verbindungen der Formel (I) und deren agrochemisch verträglichen Salze 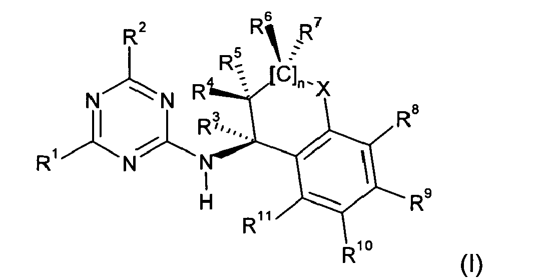 worin
R¹ und R² bedeuten unabhängig voneinander jeweils
(C₁-C₆)-Alkyl, (C₁-C₆)-Halogenalkyl, (C₁-C₆)-Halogenalkylcarbonyloxy, (C₁-C₆)-Alkoxycarbonyl-(C₁-C₄)-alkyl, (C₆-C₁₄)-Aryl-(C₁-C₆)-alkyl, (C₁-C₆)-Alkoxy-(C₁-C₆)-alkyl, (C₃-C₈)-Cycloalkyl, welches am Cycloalkylrest durch s Reste aus der Gruppe (C₁-C₆)-Alkyl und oder Halogen substituiert ist, (C₃-C₈)-Cycloalkyl-(C₁-C₆)-alkyl, (C₃-C₈)-Cycloalkyl-(C₁-C₆)-haloalkyl, (C₃-C₈)-Cycloalkenyl, (C₃-C₈)-Cycloalkenyl-(C₁-C₆)-alkyl, (C₃-C₈)-Cycloalkenyl-(C₁-C₆)-halogenalkyl, Hydroxy-(C₁-C₆)-alkyl oder Cyano-(C₁-C₆)-alkyl;
R³ bedeutet Wasserstoff, (C₁-C₆)-Alkyl oder (C₁-C₆)-Haloalkyl;
R⁴ und R⁵ bedeuten unabhängig voneinander jeweils
Wasserstoff, (C₁-C₆)-Alkyl, (C₁-C₆)-Haloalkyl, Hydroxy, (C₁-C₆)-Alkoxy, (C₁-C₆)-Halogenalkoxy, oder Benzyl, das im Phenylring durch eine Methylgruppe substituiert ist, oder die Reste R⁴ und R⁵ bilden eine (C₂-C₇)-Alkylengruppe, die ein oder mehrere Sauerstoff- und/oder Schwefelatome enthalten können, wobei die (C₂-C₇)-Alkylengruppe durch s Halogenatome substituiert ist und die jeweilgen Halogensubstituenten gleich oder verschieden sein können;
R⁶ und R⁷ bedeuten unabhängig voneinander jeweils
Wasserstoff, (C₁-C₆)-Alkyl, (C₁-C₆)-Haloalkyl, (C₁-C₆)-Alkoxy, (C₁-C₆)-Halogenalkoxy, (C₆-C₁₄)-Aryl, (C₆-C₁₄)-Aryloxy, (C₆-C₁₄)-Arylcarbonyl und (C₆-C₁₄)-Aryloxycarbonyl;
oder die Reste R⁶ und R⁷ bilden eine (C₂-C₇)-Alkylengruppe, die ein oder mehrere Sauerstoff- und/oder Schwefelatome enthalten können, wobei die (C₂-C₇)-Alkylengruppe durch s Halogenatome substituiert ist und die jeweilgen Halogensubstituenten gleich oder verschieden sein können;
R⁸, R⁹, R¹⁰ und R¹¹ bedeuten unabhängig voneinander jeweils
Wasserstoff, Halogen, Cyano, (C₁-C₆)-Alkyl, (C₁-C₆)-Alkylcarbonyl, (C₁-C₆)-Alkyloxycarbonyl, (C₁-C₆)-Alkylaminocarbonyl, (C₁-C₆)-di-Alkylaminocarbonyl, (C₁-C₆)-Haloalkyl, (C₁-C₆)-Alkoxy, (C₁-C₆)-Halogenalkoxy, (C₂-C₆)-Alkinyl, (C₂-C₆)-Halogenalkinyl, (C₂-C₆)-Alkinylcarbonyl, (C₂-C₆)-Haloalkinylcarbonyl, (C₂-C₆)-Alkinyloxy, (C₂-C₆)-Haloalkinyloxy, (C₂-C₆)-Alkinyloxycarbonyl, (C₂-C₆)-Halogenalkinyloxycarbonyl, (C₁-C₆)-di-Alkylamino, Aminocarbonyl, Cyano-(C₁-C₆)-alkyl, (C₁-C₆)-Alkyloxy-(C₁-C₆)-alkyl, (C₁-C₆)-Alkyl-(C₂-C6)-alkinyl, Methoxymethyl-(C₂-C₆)-alkinyl, Trimethylsilyl-(C₂-C₆)-alkinyl oder Nitro;
R¹² , R¹³ und R¹⁴ bedeuten unabhängig voneinander jeweils Wasserstoff, (C₁-C₆)-Alkyl oder (C₁-C₆)-Haloalkyl;
X bedeutet eine Bindung, CH₂, O, S, Carbonyl, NH, CR¹²R¹³ oder NR¹⁴;
n bedeutet 1, 2 oder 3, und
s bedeutet 0, 1, 2, 3, 4 oder 5.

2. Verbindungen nach Anspruch 1, worin
R¹ und R² bedeuten unabhängig voneinander jeweils
(C₁-C₆)-Alkyl, (C₁-C₆)-Halogenalkyl, (C₁-C₆)-Alkoxycarbonyl-(C₁-C₄)-alkyl, (C₁-C₆)-Alkoxy-(C₁-C₆)-alkyl oder (C₃-C₈)-Cycloalkyl;
R³ bedeutet Wasserstoff oder Methyl;
R⁴ und R⁵ bedeuten unabhängig voneinander jeweils
Wasserstoff, (C₁-C₆)-Alkyl, (C₁-C₆)-Alkoxy oder Benzyl, das im Phenylring durch eine Methylgruppe substituiert ist, oder die Reste R⁴ und R⁵ bilden eine (C₂-C₇)-Alkylengruppe, die ein oder mehrere Sauerstoff- und/oder Schwefelatome enthalten können, wobei die (C₂-C₇)-Alkylengruppe durch s Halogenatome substituiert ist und die jeweilgen Halogensubstituenten gleich oder verschieden sein können;
R⁶ und R⁷ bedeuten unabhängig voneinander jeweils
Wasserstoff oder (C₁-C₆)-Alkyl;
R⁸ und R⁹ bedeuten unabhängig voneinander jeweils
Wasserstoff, (C₁-C₆)-Alkyl, (C₁-C₆)-Alkoxy, Cyano oder Halogen;
R¹⁰ bedeutet Wasserstoff, Halogen, Cyano, (C₁-C₆)-Alkyl, (C₁-C₆)-Alkylcarbonyl, (C₁-C₆)-Alkoxy, (C₂-C₆)-Alkinyl, (C₁-C₆)-di-Alkylamino, Aminocarbonyl, (C₁-C₆)-Alkylaminocarbonyl, (C₁-C₆)-di-Alkylaminocarbonyl, Cyano-(C₁-C₆)-alkyl, (C₁-C₆)-Alkyloxy-(C₁-C₆)-alkyl, (C₁-C₆)-Halogenalkoxy, (C₁-C₆)-Alkyl-(C₂-C₆)-alkinyl, Methoxymethyl-(C₂-C₆)-alkinyl, Trimethylsilyl-(C₂-C₆)-alkinyl oder Nitro;
R¹¹ bedeutet Wasserstoff oder (C₁-C₆)-Alkyl;
R¹², R¹³ und R¹⁴ bedeuten unabhängig voneinander jeweils Wasserstoff oder (C₁-C₆)-Alkyl;
X bedeutet eine Bindung, Sauerstoff, Schwefel, CH₂, CR¹²R¹³ oder NR¹⁴;
n bedeutet 1, 2 oder 3, und
s bedeutet 0, 1, 2 oder 3.

3. Verbindungen nach Anspruch 1 oder 2, worin
R¹ bedeutet Methyl, Ethyl, Propyl, i-Propyl, c-Propyl, t-Butyl oder Trichlormethyl;
R² bedeutet Methyl, Ethyl, Propyl, i-Propyl, c-Propyl, t-Butyl, Trihalogenmethyl, Dihalogenmethyl, Halogenethyl, Methoxymethyl oder Methoxycarbonylmethyl;
R³ bedeutet Wasserstoff oder Methyl;
R⁴ bedeutet
Wasserstoff, Methyl, Ethyl, Methoxy oder Benzyl, das im Phenylring durch eine Methylgruppe substituiert ist,
R⁵ bedeutet Wasserstoff, Methyl oder Ethyl,
oder die Reste R⁴ und R⁵ bilden die Gruppe CH₂CH₂,
R⁶ und R⁷ bedeuten unabhängig voneinander jeweils
bedeutet Wasserstoff, Methyl oder Ethyl,
R⁸ bedeutet Wasserstoff, Methyl, Ethyl, Halogen, Cyano oder Methoxy;
R⁹ bedeutet Wasserstoff, Halogen, Cyano oder Methoxy;
R¹⁰ bedeutet Wasserstoff, Halogen, Cyano, (C₁-C₆)-Alkyl, (C₁-C₆)-Alkylcarbonyl, (C₁-C₆)-Alkoxy, (C₂-C₆)-Alkinyl, (C₁-C₂)-di-Alkylamino, Aminocarbonyl, (C₁-C₂)-Alkylaminocarbonyl, (C₁-C₆)-di-Alkylaminocarbonyl, Cyano-(C₁-C₆)-alkyl, (C₁-C₂)-Alkyloxy-(C₁-C₂)-alkyl, (C₁-C₆)-Halogenalkoxy, (C₁-C₆)-Alkyl-(C₂-C₄)-alkinyl, Methoxymethyl-(C₂-C₄)-alkinyl, Trimethylsilyl-(C₂-C₄)-alkinyl oder Nitro;
R¹¹ bedeutet Wasserstoff Methyl;
R¹² und R¹³ bedeuten unabhängig voneinander jeweils Wasserstoff, Methyl oder Ethyl;
R¹⁴ bedeutet Methyl oder Ethyl;
X bedeutet eine Bindung, Sauerstoff, Schwefel, CH₂, CR¹²R¹³ oder NR¹⁴;
n bedeutet 1, und
s bedeutet 0, 1, 2 oder 3.

4. Verfahren zur Herstellung von Verbindungen der allgemeinen Formel (I) 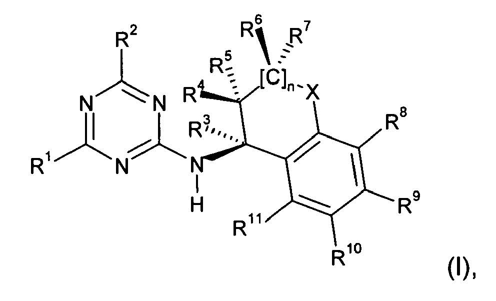 worin die genannten Reste und Indices die Bedeutungen gemäß einem der Ansprüche 1 bis 3 aufweisen, **dadurch gekennzeichnet, dass** eine Verbindung der allgemeinen Formel (II) 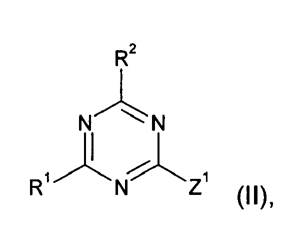 worin R¹ und R² die vorstehende Bedeutung aufweisen, und Z¹ einen austauschfähigen Rest oder eine Abgangsgruppe, wie insbesondere Chlor, Trichlormethyl, (C₁-C₄)-Alkylsulfanyl, (C₁-C₄)-Alkylsulfonyl, unsubstituiertes oder substituiertes Phenyl-(C₁-C₄)-alkylsulfonyl oder (C₁-C₄)-Alkylphenylsulfonyl, bedeutet, mit einem Amin der allgemeinen Formel (III) oder einem Säureadditionssalz davon 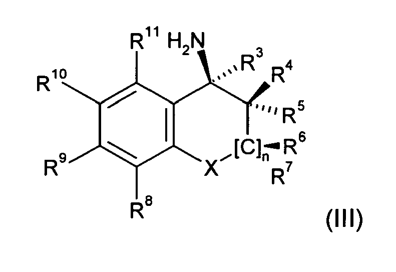 umgesetzt wird, wobei die Reste R³ bis R¹¹, n und X die vorstehende Bedeutung aufweisen.

5. Herbizides oder pflanzenwachstumsregulierendes Mittel, **dadurch gekennzeichnet, dass** es eine oder mehrere Verbindungen der Formel (I) oder deren Salze nach einem der Ansprüche 1 bis 3 enthält.

6. Herbizide Mittel nach Anspruch 5 in Mischung mit Formulierungshilfsmitteln.

7. Herbizide Mittel nach Anspruch 5 oder 6 enthaltend mindestens einen weiteren pestizid wirksame Stoffen aus der Gruppe Insektizide, Akarizide, Herbizide, Fungizide, Safenern und Wachstumsregulatoren.

8. Verfahren zur Bekämpfung von Schadpflanzen oder zur Wachstumsregulierung von Pflanzen, **dadurch gekennzeichnet, dass** man eine wirksame Menge von einer oder mehreren Verbindungen der Formel (I) oder deren Salzen nach einem der Ansprüche 1 bis 3 auf Pflanzen, Pflanzenteile, Pflanzensamen oder auf eine Anbaufläche appliziert.

9. Verwendung von Verbindungen der allgemeinen Formel (I) oder deren Salzen nach einem der Ansprüche 1 bis 3 als Herbizide oder Pflanzenwachstumsregulatoren.

10. Verwendung nach Anspruch 9, **dadurch gekennzeichnet, dass** die Verbindungen der allgemeinen Formel (I) oder deren Salze zur Bekämpfung von Schadpflanzen oder zur Wachstumsregulierung von Pflanzen in Kulturen von Nutzoder Zierpflanzen eingesetzt werden.

11. Verwendung nach Anspruch 10, **dadurch gekennzeichnet, dass** die Kulturpflanzen transgene Kulturpflanzen sind.

12. Verwendung nach Anspruch 10 oder 11, **dadurch gekennzeichnet, dass** die Kulturpflanzen aus Plantagenkulturen ausgewählt sind.
